# EUROPEAN PATENT APPLICATION

(11) **EP 4 137 512 A1**
(43) Date of publication of application: **22.02.2023**
(21) Application number: 22190485.7
(22) Date of filing: 16.08.2022
(51) Int. Cl.: C07K 16/18

(54) **ANTI-GM2AP ANTIBODY AND APPLICATIONS THEREOF**

(30) Priority: 16.08.2021 TW 110130070
(71) Applicant: Vitae Biomedical., Co., Ltd., Tapei City 115 (TW); Taiwan Innovative Integration Services Co., Ltd., Taipei City 104 (TW)
(72) Inventor: CHEN, Shui-Tein, 115 Taipei City (TW)
(74) Representative: Dörries, Hans Ulrich

(57) **Abstract**

The present disclosure disclosed herein a recombinant antibody or the antigen-binding fragment thereof which specifically binds GM2-activator protein (GM2AP). The recombinant antibody or the antigen-binding fragment thereof comprises a light chain variable region (LCVR) comprising three light chain complementary determining regions (LCDR1-3) amino acid sequences and a heavy chain variable region (HCVR) comprising three heavy chain complementary determining regions (HCDR1-3) set forth in the sequences disclosed in the embodiments of the present application. Polynucleotides encoding the same, vectors, host cells, kits and methods for detecting GM2AP and methods for inducing these recombinant antibodies or the antigen-binding fragment thereof are also provided.

## Description

### FIELD OF INVENTION

This present disclosure relates to a novel recombinant antibody or any antigen-binding fragments thereof. Specifically, the present disclosure relates to a novel recombinant antibody or any antigen-binding fragments thereof against GM2-activator protein (GM2AP) and their applications.

### SEQUENCE LISTING

This application contains a sequence listing which has been submitted in XML format complying with WIPO Standard ST.26.

### BACKGROUND OF THE INVENTION

GM2-activator protein (GM2AP) is a small monomeric protein containing a single site for Asn linked glycosylation. It is first synthesized as a precursor which is then glycosylated, modified and cleaved at ³²Ser to be in the mature form. Mature GM2AP is a glycoprotein with molecular mass of 17.6 kDa in its deglycosylated form. Acting as a cofactor, GM2AP contains at least three functional features including a hydrophobic pocket called the β-cup structure, an oligosaccharide binding site, and an area that interacts with Hex A. The area that interacts with Hex A contributes to the degradation ofGM2 ganglioside to GM3 by lysosomal β-hexminidase A (Hex A). However, only one-third of the synthesized of GM2AP is secreted. Cells can recapture the GM2AP via a carbohydrate-independent mechanism by various cells such as epidermal keratinocytes and fibroblast cells. A lack of the functional GM2AP is a cause of the abnormal accumulation ofGM2 ganglioside in tissues of patients with the AB variant of GM2 gangliosidosis disease (a severe lysosomal storage disorder). The inherited deficiency of GM2AP was also related to the changing level of ganglioside and tumor associated gangliosides involving in cancer progression. Tumor-associated gangliosides are a result of initial oncogenic transformation and play a role in the induction of invasion and metastasis. Tumor cells synthesized and shed gangliosides into their microenvironments, and this leads to elevated levels of tumor-associated gangliosides in the serum. Moreover, gangliosides are known to exhibit regulatory roles in cell growth, adhesion, cell-cell interactions and signal transduction.

### SUMMARY OF THE INVENTION

In view of the urgent need of the art, provided herein are embodiments illustrated hereinafter.

In one embodiment, the present application provides a recombinant antibody or the antigen-binding fragment thereof which specifically binds GM2-activator protein (GM2AP), wherein the recombinant antibody or the antigen-binding fragment thereof comprises (a) a light chain variable region (LCVR) comprising three light chain complementary determining regions (LCDR1-3) amino acid sequences which are independently selected from the group consisting of SEQ ID NOs: 51-53, 57-59, 63-65, 69-71, 75-77, 81-83, 87-89, 96-98, 102-104, 108-110 and 114-116; and (b) a heavy chain variable region (HCVR) comprising three heavy chain complementary determining regions (HCDR1-3) amino acid sequences which are independently selected from the group consisting of SEQ ID NOs: 54-56, 60-62, 66-68, 72-74, 78-80, 84-86, 90-92, 93-95, 99-101, 105-107, 111-113, and 117-119.

Preferably, the LCVR comprises an amino acid sequence which is selected from the group consisting of SEQ ID NOs: 5, 7, 9, 11, 13, 15, 17, 20, 22, 24 and 26, and/or the HCVR comprises an amino acid sequence which is selected from the group consisting of SEQ ID NOs: 6, 8, 10, 12, 14, 16, 18, 19, 21, 23, 25 and 27.

More preferably, the LCVR/HCVR pair comprises the amino acid sequence pairs of SEQ ID NOs: 5/6, 7/8, 9/10, 11/12, 13/14, 15/16, 17/18, 17/19, 20/21; 22/23, 24/25 or 26/27, respectively.

Preferably, the LCDR1/LCDR2/LCDR3 has an amino acid sequence of SEQ ID NOs: 51/52/53, 57/58/59, 63/64/65, 69/70/71, 75/76/77, 81/82/83, 87/88/89, 96/97/98, 102/103/104, 108/109/110 or 114/115/116, respectively.

Preferably, the HCDR1/HCDR2/HCDR3 has an amino acid sequence of SEQ ID NOs: 54/55/56, 60/61/62, 66/67/68, 72/73/74, 78/79/80, 84/85/86, 90/91/92, 93/94/95, 99/100/101, 105/106/107, 111/112/113 or 117/118/119, respectively.

More preferably, the LCDR1/LCDR2/LCDR3/HCDR1/HCDR2/HCDR3 have amino acid sequences of SEQ ID NOs: 51/52/53/54/55/56, 57/58/59/60/61/62, 63/64/65/66/67/68, 69/70/71/72/73/74, 75/76/77/78/79/80, 81/82/83/84/85/86, 87/88/89/90/91/92, 87/88/89/93/94/95, 96/97/98/99/100/101, 102/103/104/105/106/107, 108/109/110/111/112/113, or 114/115/116/117/118/119, respectively.

In any one of the foregoing embodiments, the recombinant antibody or the antigen-binding fragment thereof is selected from the group consisting of a diabody, a Fab, a Fab', a F(ab')₂, an Fv fragment, a disulfide stabilized Fv fragment (dsFv), a (dsFv)₂, a bispecific dsFv (dsfv-dsfv'), a disulfide stabilized diabody (ds diabody), a single-chain antibody molecule (scfv), an scfv dimer (bivalent diabody) and any combination thereof.

In any one of the foregoing embodiments, the recombinant antibody or the antigen-binding fragment thereof is selected from the group consisting of a monoclonal antibody, multispecific antibody, human antibody, humanized antibody, chimeric antibody and any combination thereof.

In any one of the foregoing embodiments, the recombinant antibody or the antigen-binding fragment thereof is a human antibody.

In one embodiment, the present application provides a polynucleotide encoding any one of the recombinant antibodies or the antigen-binding fragments thereof mentioned in the present disclosure. The polynucleotide comprises a nucleic acid sequence which is selected from the group consisting of SEQ ID NOs: 28-50.

Preferably, the nucleic acid sequence encoding the LCVR is selected from the group consisting of SEQ ID NOs: 28, 30, 32, 34, 36, 38, 40, 43, 45, 47 and 50, and/or the nucleic acid sequence encoding the HCVR is selected from the group consisting of SEQ ID NOs: 29, 31, 33, 35, 37, 39, 41, 42, 44, 46, 48 and 49.

In any one of the foregoing embodiments, the LCVR/HCVR is encoded by the nucleic acid sequence pair of SEQ ID NOs: 28/29, 30/31, 32/33, 34/35, 36/37, 38/39, 40/41, 40/42, 43/44, 45/46, 47/48, or 50/49, respectively.

In one embodiment, the present application provides a vector comprising any one of the polynucleotides mentioned in the present disclosure.

In one embodiment, the present application provides a host cell comprising the vector mentioned in the present disclosure.

Preferably, the host cell is selected from the group consisting of Chinese Hamster Ovary (CHO) cell, NSO cell, BHK cell, SP2/0 cell, HEK 293 cell, HEK 293 EBNA cell, PER.C6^{®} cell, COS cell, 239F cell, SF9 cell, SF21 cell and a combination thereof.

In one embodiment, the present application provides a kit for detecting a GM2-activator protein (GM2AP) in a biological sample, wherein the kit comprises the recombinant antibody or the antigen-binding fragment thereof mentioned in the present disclosure.

Preferably, the kit further comprises a substrate, and a second antibody which conjugates with a signal generating unit and binds to a Fc domain of the recombinant antibody or the antigen-binding fragment thereof. The substrate is configured to react with the signal generating unit to generate a signal.

In any one of the foregoing embodiments, the biological sample comprises whole blood, serum, plasma, urine, or a combination thereof.

In any one of the foregoing embodiments, the signal generating unit comprises a radioactive marker, a fluorescent marker, a phosphorescent marker, a chemiluminescent marker or a labeling enzyme. Preferably, the labeling enzyme may comprise a horse radish peroxidase or an alkaline phosphatase.

In any one of the foregoing embodiments, the substrate is tetramethylbenzidine (TMB) when the signal generating unit is a horse radish peroxidase.

In any one of the foregoing embodiments, the kit further comprises a blocking solution. Preferably, the blocking solution comprises skim milk, bovine serum albumin, or casein.

In any one of the foregoing embodiments, the kit further comprises a wash solution. Preferably, the wash solution comprises PBS, TBS, PBS including a detergent, or TBS including a detergent.

In one embodiment, the present application provides a method for detecting a GM2-activator protein (GM2AP) in a biological sample, comprising the steps of: (a) applying the recombinant antibody or the antigen-binding fragment thereof mentioned in the present disclosure to said biological sample; (b) incubating said biological sample with a secondary antibody conjugated with a detectable label; and (c) detecting said detectable label.

In any one of the foregoing embodiments, the biological sample comprises whole blood, serum, plasma, urine, or a combination thereof.

In any one of the foregoing embodiments, the secondary antibody binds to a Fc domain of the recombinant antibody or the antigen-binding fragment thereof.

In any one of the foregoing embodiments, the detectable label comprises an alkaline phosphatase.

In any one of the foregoing embodiments, the step (c) is conducted by incubating said detectable label with a *p*-nitrophenyl phosphate solution and determining an absorbance at OD₄₀₅.

In any one of the foregoing embodiments, the step (c) is conducted by incubating said detectable label with a tetramethylbenzidine (TMB) solution and determining an absorbance at OD₄₅₀.

In one embodiment, the present application provides a method for inducing any one of the recombinant antibody or the antigen-binding fragment thereof mentioned in the present disclosure, comprising: (a) administering to a subject a peptide fragment comprising any one of the amino acid sequences of SEQ ID NOs: 1 to 4; (b) forming hybridoma cells; and (c) screening for a hybridoma cell secreting the antibody specifically binding to the glycosylated GM2AP.

In any one of the foregoing embodiments, the peptide fragment comprises the amino acid sequence of SEQ ID NO: 1.

### BRIEF DESCRIPTION OF THE DRAWINGS

Fig. 1 illustrates a selection procedure for GM2AP monoclonal antibodies.
Fig. 2 illustrates (A) collection of the flow-through and the elution of GM2AP-His8, and (B) analysis of the flow-through the elution by coomassie blue.
Fig. 3 illustrates the analysis of the serum from the mice immunized by GM2AP by indirect ELISA.
Fig. 4 illustrates the result of the Western Blot assay staining with four anti-GM2AP antibody clones GM6, GM8, GM9 and GM10 and one commercial GM2AP antibody (Sigma).
Fig. 5 illustrates that GM9 recognizes various fragments of VITAEin protein.
Fig. 6 illustrates that GM10 recognized various fragments of VITAEin protein.

### DETAILED DESCRIPTION

The foregoing and other aspects of the present disclosure will now be described in more detail with respect to other embodiments described herein. It should be appreciated that the invention can be embodied in different forms and should not be construed as being limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

As used herein, the terms "comprises," "comprising," "includes," "including," "has", "having", "contains", "containing", "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process, or method.

The transitional phrase "consisting of' excludes any elements, steps, or ingredients not specified. If in the claim, such would close the claim to the inclusion of materials other than those recited except for impurities ordinarily associated therewith. When the phrase "consisting of' appears in a clause of the body of a claim, rather than immediately following the preamble, it limits only the element set forth in that clause; other elements are not excluded from the claim as a whole.

Where applicants have defined an invention or a portion thereof with an open-ended term such as "comprising" it should be readily understood that (unless otherwise stated) the description should be interpreted to also describe such an invention using the term "consisting of'.

As used herein, the term "about" is used to indicate that a value includes for example, the inherent variation of error for a measuring device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

The term "subject" as used herein refers to human or animals, including, for example, a mammalian subject diagnosed with or suspected of having or developing cardiovascular disease, particularly, myocardial infarction. Exemplary subject may be humans, apes, dogs, pigs, cattle, cats, horses, goats, sheep, rodents and other mammalians with the diseases that can benefit from the treatment.

The term "administering" or "administration" is referred to herein as providing an antibody of the present application to a subject. By way of example and not limitation, administration may be performed via parenteral, subcutaneous, intramuscular, intravenous, intra-articular, intrabronchial, intraabdominal, intracapsular, intracartilaginous, intracavitary, intracelial, intracerebellar, intracerebroventricular, intracolic, intracervical, intragastric, intrahepatic, ntramyocardjal, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal. For example, injection may be performed by intravenous (i.v.) injection, subcutaneous (s.c.) injection, intradermal (i.d.) injection, intraperitoneal (i.p.) injection, or intramuscular (i.m.) injection. One or more such routes may be employed.

The term "antibody" as referred to herein includes intact antibodies and any antigen- binding fragment (i.e., "antigen-binding portion") or single chains thereof. An "antibody" refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as VH) and a heavy chain constant region (abbreviated herein as CH). The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each of V_{H} and V_{L} is composed of three CDRs and four FRs, arranged from amino-terminus to carboxy-terminus in the following order: FRI, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The "epitope" or "antigenic determinant" is a portion of an antigen molecule that is responsible for specific interactions with the antigen-binding domain of an antibody. An antigen-binding domain may be provided by one or more antibody variable domains. An antigen-binding domain can comprise at least one antibody light chain variable region (VL) and at least one antibody heavy chain variable region (VH). An antigen-binding domain can also comprise only VH or only VL regions. Specifically, in the present disclosure, the epitope could be a specific domain or a combination of domains of human GM2AP. In some embodiments of the present disclosure, the specific domains or combination of domains are of the human GM2AP that comprises the amino acid sequence of SEQ ID NO: 1.

The term "complementarity determining regions" ("CDR") are defined as parts of the variable chains in antibodies, where these molecules bind to their specific antigen. In this disclosure, the CDR regions in the heavy chain are typically referred to as CDR-H1, CDR-H2 and CDR-H3 and in the light chain as CDR-L1, CDR-L2 and CDR-L3. They are numbered sequentially in the direction from the amino terminus to the carboxy terminus.

The extent of the framework regions and CDRs has been defined according to Kabat et al. (see, Kabat et al., Sequences of Proteins of Immunological Interest, U.S. Department of Health and Human Services, 1991) and the ImMunoGeneTics database (IMGT) (see, Lefranc, Nucleic Acids Res 29:207-9, 2001; and online at imgt.cines.fr/IMGT_vquest/vquest?livret=O&Option=humanlg).

The "Kabat system" means in the context of the present disclosure the standard for numbering the residues in a consistent manner according to Kabat (1991; Sequences of Proteins of Immunological Interest, 5th edit., NIH publication No. 91-3242U.S. Department of Health and Human Services) and Chothia (1987; J. Mol. Biol. 196, 901-917). This numbering system is widely used by the skilled artisans and is based on sequence variability and three dimensional loops of the variable domain region which are important in antigen-binding activity. All the residues of the light chains or heavy chains have distinct positions in the Kabat system; i.e., the Kabat system applies to CDRs as well as to framework regions. The positions of specific residues of any antibody may be numbered according to the Kabat system. The rules to identify the CDR regions of VH and VL chains according to Kabat system are shown in www.bioinf.org.uk/abs.

The IMGT unique numbering system is an alternative to the Kabat System that allows one to compare the variable domains whatever the antigen receptor, the chain type, or the species [Lefranc M.-P., Immunology Today 18, 509 (1997)/Lefranc M.-P., The Immunologist, 7, 132-136 (1999)/Lefranc, M.-P., Pomrnie, C., Ruiz, M., Giudicelli, V., Foulquier, E., Truong, L., Thouvenin-Contet, V. and Lefranc, Dev. Comp.lmmunol., 27, 55-77 (2003)]. In the IMGT unique numbering system, the conserved amino acids always have the same position, for instance cysteine 23 (1st-CYS), tryptophan 41 (CONSERVED-TRP), hydrophobic amino acid 89, cysteine 104 (2nd-CYS), phenylalanine or tryptophan 118 (J-PHE or J-TRP). The IMGT unique numbering system provides a standardized delimitation of the framework regions (FRl- IMGT: positions 1 to 26, FR2-IMGT: 39 to 55, FR3-IMGT: 66 to 104 and FR4-IMGT: 118 to 128) and of the complementarity determining regions: CDRl-IMGT: 27 to 38, CDR2-IMGT: 56 to 65 and CDR3-IMGT: 105 to 117. As gaps represent unoccupied positions, the CDR- IMGT lengths (shown between brackets and separated by dots, e.g. [8.8.13]) become crucial information. The IMGT unique numbering system is used in 20 graphical representations, designated as IMGT Colliers de Perles [Ruiz, M. and Lefranc, M.-P., Immunogenetics, 53, 857-883 (2002)/Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., Tcell receptor and MHC structural data. Nucl. Acids. Res., 32, D208-D210 (2004)].

The term "antigen-binding fragment" as used herein refers to an antibody fragment, such as for example, a diabody, a Fab, a Fab', a F(ab')₂, an Fv fragment, a disulfide stabilized Fv fragment (dsFv), a (dsFv)₂, a bispecific dsFv (dsfv-dsfv'), a disulfide stabilized diabody (ds diabody), a single-chain antibody molecule (scfv), an scfv dimer (bivalent diabody), a multispecific antibody formed from a portion of an antibody comprising one or more CDRs, or any other antibody fragment that binds to an antigen but does not comprise a complete or intact antibody structure. An antigen-binding fragment is capable of binding to the same antigen to which the parent antibody or a parent antibody fragment (e.g., a parent scFv) binds. In certain embodiments, an antigen-binding fragment may comprise one or more CDRs from a particular human antibody grafted to a framework region from one or more different human antibodies.

Among the above antigen-binding fragments, a Fab, which is a structure having the light chain and heavy chain variable regions, the light chain constant region, the heavy chain first constant region (CH1), and bas one antigen-binding site. A Fab ' differs from the Fab in that the Fab' has a hinge region including at least one cysteine residue at the C-terminal of the heavy chain CH1 domain. A F(ab')₂ is produced when cysteine residues at the hinge region of Fab' are joined by a disulfide bond.

An Fv is a minimal antibody fragment, having only heavy chain variable region and light chain variable regions. A recombinant technique for producing the Fv fragment is well known in the art. "Single-chain Fv antibody" or "scFv" refers to an engineered antibody consisting of a light chain variable region and a heavy chain variable region connected to one another directly or via a peptide linker sequence. A two-chain Fv may have a structure in which heavy chain variable regions are linked to light chain variable regions by a non-covalent bond. A single-chain Fv may generally form a dimer structure as in the two-chain Fv, wherein heavy chain variable regions are covalently bow1d to light chain variable regions via a peptide linker or the heavy and light chain variable regions are directly linked to each other at the C-terminals thereof. The linker may be a peptide linker including any 1 to 100 or 2 to 50 amino acids, and proper sequences useful therefor are well known in the art.

The antigen-binding fragment may be obtained using a protease (for example, a whole antibody can be digested with papain to obtain Fab fragments, or can be digested with pepsin to obtain F(ab')₂ fragments), or may be prepared by a genetic recombinant technique.

The terms "monoclonal antibody" or "monoclonal antibody composition" as used herein refer to a preparation of antibody molecules of homogeneous molecular composition. A monoclonal antibody composition displays a single binding specificity and affinity for a particular epitope.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the disclosure may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences.

The term "recombinant" as used herein, is intended to include all molecules that are prepared, expressed, created or isolated by recombinant means, such as multispecific molecules (e.g. bispecific molecules) expressed using a recombinant expression vector transfected into a host cell, multispecific molecules (e.g., bispecific molecules) isolated from an animal (e.g., a mouse) that is transgenic for human immunoglobulin genes (see e.g., Taylor et al. (1992) Nucl. Acids Res. 20:6287-6295) or multispecific molecules prepared, expressed, created or isolated by any other means that involves splicing of human immunoglobulin and/or MHC gene sequences to other DNA sequences. Such recombinant multispecific molecules can include antigen-binding domains having variable and constant regions derived from human germline immunoglobulin sequences.

The term "recombinant human antibody" as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the V_{H} and V_{L} regions of the recombinant human antibodies are sequences that, while derived from and related to human germline V_{H} and V_{L} sequences, may not naturally exist within the human antibody germline repertoire in vivo.

The term "humanized antibody" is referred to as an antibody that is generated from non-human species and comprises protein sequences that have been modified to increase their similarity to antibody variants produced naturally in humans. The humanization process could be necessary to avoid undesired immunogenic effect when applying a non-human source antibody in human. In comparison, the term "chimeric antibody" as referred to herein is an antibody made by fusing the antigen binding region (i.e. VH and VL) from one species with the constant domain with another.

The term "K_{assoc}" or "Kₐ," as used herein, is intended to refer to the association rate of a particular antibody-antigen interaction, whereas the term "K_{dis}" or "K_{d}" as used herein, is intended to refer to the dissociation rate of a particular antibody-antigen interaction. The term "K_{D}" as used herein, is intended to refer to the dissociation constant, which is obtained from the ratio of K_{d} to Kₐ (i.e., K_{d}/Kₐ) and is expressed as a molar concentration (M). K_{D} values for antibodies can be determined using methods well established in the art. Preferably, the K_{D} values are determined in a Surface plasmon resonance (SPR) assay using, e.g., BIAcore such as a GE BIAcore 3000 instrument.

The terms "percent (%) sequence identity" or "homology" are defined as the percentage of amino acid residues or nucleotides in a candidate sequence that are identical with the amino acid residues or nucleotides in the reference sequences after aligning the sequences and introducing gaps, if necessary, to achieve the maximum percent sequence identity, and excluding conservative nucleic acid substitutions. Optimal alignment of the sequences for comparison may be produced, besides manually, by means of local homology algorithms known in the art or by means of computer programs which use these algorithms (e.g., BLAST P).

The nucleic acid sequences and the amino acid sequences mentioned in the following embodiments are summarized in Tables 1-3.

**Table 1. Amino Acid Sequences of GM2AP**

| SEQ ID NO: | Designation | Sequences |
|---|---|---|
| 1 | GM2AP fragment | PIIVPGNVTLSVVG |
| 2 | GM2AP | |
| 3 | GM2AP | |
| 4 | GM2AP | MQSLMQAPLLIALGLLLAAPAQAHLKKPSQLSSFSWDNCDEGKDPAVIRSLT LEPDPIIVPGNVTLSVMGSTSVPLSSPLKVDLVLEKEVAGLWIKIPCTDYIGSC TFEHFCDVLDMLIPTGEPCPEPLRTYGLPCHCPFKEGTYSLPKSEFVVPDLELP SWLTTGNYRIESVLSSSGKRLGCIKIAASLKGIRHHHHHHHH |

**Table 2. Amino Acid Sequences of the recombinant antibodies exemplified by the present application**

| SEQ ID NO: | Designation | Sequences **(Bold:** CDR1; *Italic*: CDR2; 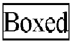 : CDR3) |
|---|---|---|
| 5 | 21-2B5-F7 LCVR | DIVMTQTASSVYVTPGESVSISCRSTKSLLHSNGDTYLYWFLQRPGQSPQLLI YRMSNLAPGVPDRFSGGGSGTAFTLRISRVEAEDVGVYYLYATSTISFHVRC WDQAGAET |
| 6 | 21-2B5-F7 HCVR | EIQLQQSGAELVRPGTSVKISCMASGYSFTGYNMNWVRQSHGRSLEWIGNIN PYFGSPNYNQKFKGKATLTLDRSSNTAYMQLNRVTSEDSAVYYCASRQLGL GDTMDYWGQGTSVTVSS |
| 7 | 21-4E3-C8 LCVR (mixed clone) | DIVMTQSHKFMSTSVGDRVTITCKASQDVSTAVGWYQQKPGQSPKLLIYWA STRHTGVPDRFTGSRFGTDYTLTISSVQAEDLALYYCQQHYITPWTFGGGTK LEIK |
| 8 | 21-4E3-C8 HCVR (mixed clone) | QVQLQQPGADFVKPGASVKLSCKASGYTFTSYWINWVKQRPGQGLEWIGNI YPGSGSTNYNEKFKNKATLTIDTSSSTAYMQLSSLTSDDSAVYYCSRSTLYSY DDGSWGQGTTLTVSS |
| 9 | 21-4E3-C8 LCVR (mixed clone) | DIQMTQSPSSLSASLGERVSLTCRASQEISGDLSWLQQKPDGTIKRLIYAASTL ASGVPKRFSGSRSGSDYSLTISGLESEDFADYYCLQYVSYPFTFGSGTKLEIK |
| 10 | 21-4E3-C8 HCVR (mixed clone) | QIQLQQSGPELGKPGASVKISCKASDYTFTDFYISWVKQRPGQGLEWIGWIYP LDDHTKYNEKFKGKATLTVDISSNTVYMQLSSLTSEDSAVYFCARIYGYAMD YWGQGTSVTVSS |
| 11 | 21-2H9-G2 LCVR | DIQMTQSPSSLSASLGERVSLTCRASQEISGDLSWLQQKPDGTIKRLIYAASTL ASGVPKRFSGSRSGTDYSLTISGLESEDFADYYCLQYVSYPFTFGSGTKLEIK |
| 12 | 21-2H9-G2 HCVR | QVQLQQPGADFVKPGASVKLSCKASGYTFTSYWINWVKQRPGQGLEWIGNI YPGSGSTNYNEKFKNKATLTIDTSSSTAYMQLSSLTSDDSAVYYCSRSTLYSY DDGSWGQGTTLTVSS |
| 13 | 21-4G4-B11 LCVR | EIVMTQSHNHVHISGRQGHHHLQGQSGCEYCCRLVSTETRAISITTDFTGHPP GTLESLIAFTGSRSGTDYTLTISSVQAEDLALYYCQQHYITPWTFGGGTKLEIK |
| 14 | 21-4G4-B11 HCVR | QIQLQQSGPELGKPGASVKISCKASDYTFTDFYISWVKQRPGQGLEWIGWIYP LDDHTKYNEKFKGKATLTVDISSNTVYMQLSSLTSEDSAVYFCARIYGYAMD YWGQGTSVTVSS |
| 15 | 21-4B10-E1 LCVR | DIVMTSETHLCICHSWRVSLHLLQVYIESPAIIWRHLFVLVPTEARPVSSAPDI SDVQPCPRSPTQVQWRWVRNCFHTENQISGGTGCGCLLLYATSTISFHVRCW DQAGAET |
| 16 | 21-4B10-E1 HCVR | EIQLQQSGAELVRPGTSVKISCMASGYSFTGYNMNWVRQSHGRSLEWIGNIN PYFGSPNYNQKFKGKATLTLDRSSNTAYMQLNRVTSEDSAVYYCASRQLGL GDTMDYWGQGTSVTVSS |
| 17 | 22-2E9-G12 LCVR (mixed clone) | DIVMTQSQKFMSTSIGDRVSVTCKASQNVNTNIAWYQQKPGQSPKALIYSAS YRYSGVPDRFTGSGSGTDFTLTISSVQSEDLAEYFCQQYIGYPYTFGGGTKLEI K |
| 18 | 22-2E9-G12 HCVR (mixed clone) | QVQLQQPGATLVRPGASVKLSCKASGYTFINYWINWVKWRPGQGLEWIGNI NPNCGVTKYNEQIKSEATLTVDKSSSIAYMQLSSLTSEDSAVYYCPHLLLRWL LHVPFAMVYWGQGTSV |
| 19 | 22-2E9-G12 HCVR (mixed clone) | EVQLVETGGGLVQPQGSLKLSCAASGFNFNTYAMNWVRQAPGKGLEWVA RIRTKSNSYATYYADSVKDRFIISRDDSQSMLYLQMNNLKTEDTAIFYCVVTY WGFIYWGQGTLVTVSA |
| 20 | 22-3E6-B11 LCVR | DVVMTQTPLTLSVTIGQPASISCKSSQSLLYSDRKTYLNWLFQRPGQSPKRLI YLVSKLDSGVPDRFTGSGSGTDFTLKISRVEAEDLGIYYCWQGTHFPQTFGGG TKLEIK |
| 21 | 22-3E6-B11 HCVR | EVQLQQSGPELVKPGASVKMSCKASGYSFTAYYIHWMKQSPEKSLEWIGEIN PSTDDTTYNQNFKSKATLTVDRSSRTAYMQLRSLTSEDSAVYFCARYGEGAY WGQGTLVTVSA |
| 22 | 22-4B11-B9 LCVR | DVVMTQIPLTLSVIIGQPASISCKSSQSLLYSDGKTYLNWLLQRPGQSPKRLIY LVSKLDSGVSDRFTGSGSGTDFTLKISRVEAEDLGVYYCWQGTHFPQTYGGG TKLEIK |
| 23 | 22-4B11-B9 HCVR | EVQLQQSGPELVKPGASVKISCKASGYSFTGFPHALGEAKSTKEPTVDWRDI SIYWWYYLQSEVQGQGHIDCRQILQYSLYATQEPDITRLCSLLLCKIRRGGLLG PRDSGLCLC |
| 24 | 22-5B1-B2 LCVR | IVMTQSPKSMSMSVGERVTLSCKASENVGTYVSWYQQKPEQSPKLLIYGASN RYTGVPDRFTGSGSATDFTLTISSVQAEDLADYHCGQSYSYPLTFGAGTKLEL K |
| 25 | 22-5B1-B2 HCVR | EVQMQQSGPELVKPGASVEISCKASGYSFTGYYIHWVKQSPEKNLEWIGEVN PISGGTTYNQRFKAKATLNVDTLSNTAYMQLKSLTSEDSAVYFCARYGATD YWGQGTSVTVSS |
| 26 | 22-5F2-E10 LCVR | EVQLQQSGPELVKPGASVKISCKASGYSFTGYYIHWVRRSPEKSLAWIGEINP TTGGTSYNQKFRAKATLTVDTSSSTAYIQLKSLTSEDSAIYYCARYGEGAYW GQGTLVTVSA |
| 27 | 22-5F2-E10 HCVR | DVVLTQTPLTLSVTFGQPASISCKSSQSLFIITWKDIFVFGCYRGQASLQSAIS FWCLNWTLESLTGSVAVDQGQISHTRSTEWRLRIWEFIIAGKVHIFLRRTVEA PCWKSN |

**Table 3. Nucleic Acids Sequences encoding the LCVR/HCVR of the recombinant antibodies exemplified by the present application.**

| SEQ ID NO: | Designation | Sequences |
|---|---|---|
| 28 | 21-2B5-F7 LCVR | gttagatctgtgagcgagctgcgtagaatgagttcctggcgctgcttgtgctctggatccctggagccattggggatattgtg atgactcagactgcatcctctgtatatgtcactcctggagagtcagtctccatctcctgcaggtctactaagagtctcctgcat agtaatggcgacacttatttgtattggttcctacagaggccaggccagtctcctcagctcctgatatatcggatgtccaacctt gccccaggagtccctgacaggttcagtggcggtgggtcaggaactgctttcacactgagaatcagtagagtggaggctga ggatgtgggtgtttattacctgtatgcaacatcttgaatatcctttcacgttcggtgctgggaccaagctggagctgaaacgg gctgatgttgcaccaactgtatccatcttcccaccatccagtgagcagttaacatctggaggtgcctcagtcgtgtgcttcttg aacaacttctaccccaaagacatcaatgtcaagtggaagattgatggcagtgaacgacaaaatggcgtcctgaacagttgg actgatcaggacagcaaagacagcacctacagcatgagcagcaccctcacgttgaccaaggacgagtatgaacgacata acagctatacctgtgaggccactcacaagacatcaacttcaccccattgtcaagagcttcaacaggatgagtgtacgcg |
| 29 | 21-2B5-F7 HCVR | tacaaagtaaccactcaaaggataaaataatcgcgaatttgccccccccaagagacaacgacgaagattggggtcatcct cttccttctgtcagtaactgcaggtgtccactctgagatccagctgcaacagtctggagctgagctggtgaggcctgggact tcagtgaagatatcctgcatggcttctggttactcattcactggctacaatatgaactgggtgaggcaaagccatggaagga gccttgagtggatcggaaatattaatccttactttggtagtcctaactacaatcagaagttcaagggcaaggccacattgactt tagacagatcctccaacacagcctacatgcagctcaacagagtgacatctgaggactctgcagtctactactgtgcatcaa ggcagctcgggctgggagatactatggactactggggtcaaggaacctcagtcaccgtctcctcagccaaaacaacagc cccatcggtctatccactggcccctgtgtgtggagatacaagtggctcctcggtgactctaggatgcctggtcaagggttatt tccctgagccagtgaccttgacctggaactctggatccctgtccagtggtgtgcacaccttcccagctgtcctgcagtctga cctctacaccctcagcagctcagtgactgtaacctcgagcacctggcccagccagtccatcacctgcaattgtcgct |
| 30 | 21-4E3-C8 LCVR (mixed clone) | gtgtttctccggttgtctggtgttgacggagacattgtgatgacccagtctcacaaattcatgtccacatcagtgggagacag ggtcaccatcacctgcaaggccagtcaggatgtgagtactgctgtaggctggtatcaacagaaaccagggcaatctccta aactactgatttactgggcatccacccggcacactggagtccctgatcgcttcacaggcagtagatttgggacagactatac tctcaccatcagcagtgtgcaggctgaagacctggcactttattactgtcagcaacattatatcactccgtggacgttcggtg gaggcaccaagctggaaatcaaacgggctgatgctgcaccaactgtatccatcttcccaccatccagtgagcagttaacat ctggaggtgcctcagtcgtgtgcttcttgaacaacttctaccccaaagacatcaatgtcaagtggaagattgatggcagtga acgacaaaatggcgtcctgaacagttggactgatcaggacagcaaagacagcacctacagcatgagcagcaccctcac gttgaccaaggacgagtatgaacgacataacagctatacctgtgaggccactcacaagaccacaacttcacccattcaac gg |
| 31 | 21-4E3-C8 HCVR (mixed clone) | cgacagtctttttggttgcagcagctacaggtgtccactcccaggtccaactgcagcagcctggtgctgactttgtgaagcct ggggcctcagtgaagctgtcctgcaaggcttctggctacactttcaccagctactggataaactgggtgaagcagaggcct ggacaaggccttgagtggattggaaatatttatcctggtagcggtagtactaactacaatgagaagttcaagaacaaggcc acactgactatagacacatcctccagcacagcctacatgcagctcagcagcctgacatctgacgactctgcggtctactact gttcaagatcaaccctctatagttacgacgacggctcttggggccaaggcaccactctcacagtctcctcagccaaaacga cacccccatctgtctatccactggcccctggatctgctgcccaaactaactccatggtgaccctgggatgcctggtcaagg gctatttccctgagccagtgacagtgacctggaactctggatccctgtccagcggtgtgcacaccttcccagctgtcctgca gtctgacctctacactctgagcagctcagtgactgtcccctccagcacctggcccagcgagaccgtcacctgcaacgtgc ccctccc |
| 32 | 21-4E3-C8 LCVR (mixed clone) | tgttgctctggtttccaggtatcagatgtgacatccagatgacgcagtctccatcctccttatctgcctctctgggagaaagag tcagtctcacttgtcgggcaagtcaggaaattagtggtgacttaagctggcttcagcagaaaccagatggaactattaaacg cctgatatacgccgcatccactttagcttctggtgtcccaaaaaggttcagtggcagtaggtctgggtcagattattctctcac catcagcggccttgagtctgaagattttgcagactattactgtctacaatatgttagttatccattcacgttcggctcggggaca aagttggaaataaaacgggctgatgctgcaccaactgtatccatcttcccaccatccagtgagcagttaacatctggaggtg cctcagtcgtgtgcttcttgaacaacttctaccccaaagacatcaatgtcaagtggaagattgatggcagtgaacgacaaaa tggcgtcctgaacagttggactgatcaggacagcaaagacagcacctacagcatgagcagcaccctcacgttgaccaag gacgagtatgaacgacataacagctatacctgtgaggccacttcacatagacagcaaatctcacccattcaacag |
| 33 | 21-4E3-C8 HCVR (mixed clone) | ttgaacttgccattcctcctgtcaggaactgcaggtgtccattgccagatccagctgcagcagtctggacctgaactgggga agcctggggcttcagtgaagatatcctgcaaggcttctgactacaccttcactgacttctatataagttgggtgaaacagagg cctggacagggacttgagtggattggatggatttatcctttagacgatcatactaagtacaatgagaagttcaagggcaagg ccacattgactgtagacatatcctccaacacagtctacatgcagctcagcagcctgacctctgaggactctgcggtctatttc tgtgccagaatctatggatatgctatggactactggggtcaaggaacctcagtcaccgtctcctcagccaaaacaacagcc ccatcggtctatccactagcccctgtgtgtggagatacaactggctcctcggtgactttaggatgcctggtcaagggttatttc cctgagccagtgaccttgacctggaactctggattcctgtccagtggtgtgcacacctttccagctgtcctgcagtctgacct ctacaccctcagcagctcagcgactgtaacctcgagcacctggcccagccataccatacctgcagacggtccccc |
| 34 | 21-2H9-G2 LCVR | gcggtcatctgcacgatcatgagggtacctcgctcacgtgctttaagattcttgtcagctctggtttccaggtatcagatgtga catccagatgacccagtctccatcctccttatctgcctctctgggagaaagagtcagtctcacttgtcgggcaagtcaggaa attagtggtgacttaagctggcttcagcagaaaccagatggaactattaaacgcctgatctacgccgcatccactttagcttct ggtgtcccaaaaaggttcagtggcagtaggtctgggtgagattattctctcaccatcagcggccttgagtctgaagattttgc agactattactgtctacaatatgttagttatccattcacgttcggctcggggacaaagttggaaataaaacgggctgatgctgc accaactgtatccatcttcccaccatccagtgagcagttaacatctggaggtgcctcagtcgtgtgcttcttgaacaacttcta ccccaaagacatcaatgtcaagtggaagattgatggcagtgaacgacaaaatggcgtcctgaacagttggactgatcagg acagcaaagacagcacctacagcatgagcagcaccctcacgttgaccaaggacgagtatgaacgacataacagctatac ctgtgaggccactcacaagacatcagttttcccccattatcagagggctttcaccaggaatgagtgtagcccgccaaaaaa aaaaaaaaac |
| 35 | 21-2H9-G2 HCVR | cgtctgtctttttggttgcagcagctacaggtgtccactcccaggtccaactgcagcagcctggtgctgactttgtgaagcct ggggcctcagtgaagctgtcctgcaaggcttctggctacactttcaccagctactggataaactgggtgaagcagaggcct ggacaaggccttgagtggattggaaatatttatcctggtagcggtagtactaactacaatgagaagttcaagaacaaggcc acactgactatagacacatcctccagcacagcctacatgcagctcagcagcctgacatctgacgactctgcggtctactact gttcaagatcaaccctctatagttacgacgacggctcttggggccaaggcaccactctcacagtctcctcagccaaaacga cacccccatctgtctatccactggcccctggatctgctgccccaaaactaactccatggtgaccctgggatgcctggtcaag ggctatttccctgagccagtgacagtgacctggaactctggatccctgtccagcggtgtgcacaccttcccagctgtcctgc agtctgacctctacactctgagcagctcagtgactgtcccctccagcacctggcccagcgagaccgtcacctgcaacgtg cccgtcca |
| 36 | 21-4G4-B11 LCVR | gactagagcacgtggtgtctggtgttgacggagaaattgtgatgacccagtctcacaatcatgtccacatcagtgggagac agggtcaccatcacctgcaaggccagtcaggatgtgagtactgctgtaggctggtatcaacagaaaccagggcaatctcc taaactactgattttactgggcatccacccggcacactggagtccctgatcgctttcacaggcagtagatctgggacagact atactctcaccatcagcagtgtgcaggctgaagacctggcactttattactgtcagcaacattatatcactccgtggacgttcg gtggaggcaccaagctggaaatcaaacgggctgatgctgcaccaactgtatccatcttcccaccatccagtgagcagttaa catctggaggtgcctcagtcgtgtgcttcttgaacaccttctaccccaaagacatcaatgtcaagtggaagattgatggcagt gaacgacaaaatggcgtcctgaacagttggactgatcaggacagcaaagacagcacctacagcatgagcagcaccctc acgttgaccaaggacgagtatgaacgacataacagctatacctgtgaggccactcacaagacatcaacttcacccattgtc aagagcttcaacaggaatgagtgtagggggggggaaaaaaaaaaaaaaaaaaaaaaggcggaggggacgcggataa aaataaccgttagcatgacttaagtgcgtaacatattcaatctttctttccgcgggggcgggaaagagg |
| 37 | 21-4G4-B11 HCVR | catgaatggagctgggtcattctcttcctcctgtcaggaactgcaggtgtccattgccagatccagctgcagcagtctggac ctgaactggggaagcctggggcttcagtgaagatatcctgcaaggcttctgactacaccttcactgacttctatataagttgg gtgaaacagaggcctggacagggacttgagtggattggatggatttatcctttagacgatcatactaagtacaatgagaagt tcaagggcaaggccacattgactgtagacatatcctccaacacagtctacatgcagctcagcagcctgacctctgaggact ctgcggtctatttctgtgccagaatctatggatatgctatggactactggggtcaaggaacctcagtcaccgtctcctcagcc aaaacaacagccccatcggtctatccactggcccctgtgtgtggagatacaactggctcctcggtgactctaggatgcctg gtcaagggttatttccctgagccagtgaccttgacctggaactctggatccctgtccagtggtgtgcacaccttcccagctgt cctgcagtctgacctctacaccctcagcagctcagtgactgtaacctcgagcacctggcccagccagtccatcacctgcaa tggcccgcag |
| 38 | 21-4B10-E1 LCVR | gcccccccttttttatgggggggggaacttgcctcagctcagttccttgggctgcttgtgctctggatccctggagccattggg gatattgtgatgacttcagaaactcacctctgtatatgtcactcctggagagtcagtctccatctcctgcaggtctactaagagt ctcctgcatagtaatggcgacacttatttgtattggttcctacagaggccaggccagtctcctcagctcctgatatatcggatg tccaaccttgccccaggagtccctgacaggttcagtggcggtgggtcaggaactgctttcacactgagaatcagtagagtg gaggctgaggatgtgggtgtttattactgtatgcaacatcttgaatatcctttcacgttcggtgctgggaccaagctggagctg aaacgggctgatgttgcaccaactgtatccatcttcccaccatccagtgagcagttaacatctggaggtgcctcagtcgtgt |
| 39 | 21-4B10-E1 HCVR | agccttccccccccaaagggaggggtgaccttgctattccttctgtcagtaactgcaggtgtccactctgagatccagctgc aacagtctggagctgagctggtgaggcctgggacttcagtgaagatatcctgcatggcttctggttactcattcactggctac aatatgaactgggtgaggcaaagccatggaaggagccttgagtggatcggaaatattaatccttactttggtagtcctaacta caatcagaagttcaagggcaaggccacattgactttagacagatcctccaacacagcctacatgcagctcaacagagtga catctgaggactctgcagtctactactgtgcatcaaggcagctcgggctgggagatactatggactactggggtcaaggaa cctcagtcaccgtctcctcagccaaaacaacagccccatcggtctatccactagcccctgtgtgtggagatacaagtggct cctcggtgactttaggatgcctggtcaagggttatttccctgagccagtgaccttgacctggaactctggatccctgtccagt ggtgtgcacaccttcccagctgtcctgcagtctgacctctacaccctcagcagctcagtgactgtaacctcgagcacctggc ccagctcagtccgtcgcactgcttgctgttca |
| 40 | 22-2E9-G12 LCVR (mixed clone) | ggtcatactgctgtggtgtctggtgttgatggagacattgtgatgacccagtctcaaaaattcatgtccacatcaataggaga cagggtcagcgtcacctgcaaggccagtcagaatgtgaatactaatatagcctggtatcaacagaaaccaggacagtctc ctaaagcactgatttactcggcttcctaccgctatagtggagtccctgatcgcttcacaggcagtggatctgggacagatttc actctcaccatcagcagtgtgcagtctgaggacttggcagaatatttctgtcagcaatatatcggctatccgtacacgttcgg aggggggaccaagctggaaataaaacgggctgatgctgcaccaactgtatccatcttcccaccatccagtgagcagttaa catctggaggtgcctcagtcgtgtgcttcttgaacaacttctaccccaaagacatcaatgtcaagtggaagattgatggcagt gaacgacaaaatggcgtcctgaacagttggactgatcaggacagcaaagacagcacctacagcatgagcagcaccctc acgttgaccaaggacgagtatgaacgacataacagctatacctgtgaggccactcacaagacatcaacttcacccattgtc aagagcttcaacaggaatgagtgtaaccc |
| 41 | 22-2E9-G12 HCVR (mixed clone) | gggtcatctcttcttggtagcaactgctacaggtgtccactcccaggtccaactgcagcagcctggggcttgactggtgag gcctggggcttcagtgaagttgtcctgcaaggcttctggctacaccttcatcaactactggataaactgggtgaagtggagg cctggacaaggccttgagtggattggaaatattaatcctaattgtggtgttactaagtacaatgagcagatcaagagcgagg ccacactgactgtagacaaatcctccagcatagcctacatgcaactcagcagcctgacatctgaggactctgcggtctatta ctgtcctcatttattactacgatggttgctacacgtcccctttgctatggtctactggggtcaaggaacctcagtcaccgtctcc tcagccaaaacaacagccccatcggtctatccactggcccctgtgtgtggagatacaagtggctcctcggtgactctagga tgcctggtcaagggttatttccctgagccagtgaccttgacctggaactctggatccctgtccagtggtgtgcacaccttccc agctgtcctgcagtctgacctctacaccctcagcagctcagtgactgtaacctcgagcacctggcccagccagtccatcac ctgcaattggccctcccca |
| 42 | 22-2E9-G12 HCVR (mixed clone) | gtggggtcaatatacattgtcatgctgttagggctgaagtgggttttctttgttgttttttatcaaggtgtgcattgtgaggtgcag cttgttgagactggtggaggattggtgcagcctcaagggtcattgaaactctcatgtgcggcctctggattcaacttcaatac ctatgccatgaactgggtccgccaggctccaggaaagggtttggaatgggttgctcgcataagaactaaaagtaattcttat gcaacatattatgccgattcagtgaaagacaggttcatcatctccagagatgattcccaaagcatgctctatctgcaaatgaa caacttgaaaactgaggacacagccatattttactgtgtggttacctactgggggtttatttactggggccaagggactctggt cactgtctctgcagccaaaacaacagccccatcggtctatccactggcccctgtgtgtggagatacaagtggctcctcggt gactctaggatgcctggtcaagggttatttccctgagccagtgaccttgacctggaactctggatccctgtccagtggtgtgc acaccttcccagctgtcctgcagtctgacctctacaccctcagcagctcagtgactgtaacctcgagcacctggcccagcc agtccatcacctgcaatgtggcccaacacggcaagcagcacccgtaaaaaaaaaaatttggt |
| 43 | 22-3E6-B11 LCVR | agctcaacaacaaaaaaaacaaaaaacaaaaaaagataaaatctacacgtgacgggagttggggggagaggcagtgct cttttttttaggggggggggacactagttgcctgttaggctgttggtgctctggattcgggaaaccaacggtgatgttgtgatg acccagactccactcactttgtcggttaccattggacaaccagcctccatatcttgcaagtcaagtcagagcctcttatatagt gatagaaagacatatttgaattggttgtttcagaggccaggccagtctccaaagcgcctaatctatctggtctctaaactgga ctctggagtccctgacaggttcactggcagtggatcagggacagatttcacactgaaaatcagcagagtggaggctgagg atttgggaatttattattgctggcaaggtacacattttcctcagacgttcggtggaggcaccaagttggaaatcaaacgggct gatgctgcaccaactgtatccatcttcccaccatccagtgagcagttaacatctggaggtgcctcagtcgtgtgcttcttgaa caacttctaccccaaagacatcaaagtcaaagtggaagattgatggcagtgaacgacaaaatggcgtcctgaacagttgg actgatcaggacagcaaagacagcacctacagcatgagcagcaccctcacgttgaccaaggacgagtatgaacgacata acagctatacctgtgaggccactcacaaagagatcaaatttcacccattcaacg |
| 44 | 22-3E6-B11 HCVR | gtaggaagctgggtttcctcttaatcctgtcagtaactacaggtgtcccactctgaggtccagctgcagcagtctggacctga gctggtgaagcctggggcttcagtgaagatgtcctgcaaggcttctggttactcattcactgcctattacatacactggatga agcaaagtcctgaaaagagccttgagtggattggagagattaatcctagcactgatgacacaacctacaaccagaacttca agtccaaggccacattgactgtagacaggtcctccagaacagcctacatgcaactcaggagcctgacatctgaggactct gcagtctatttctgtgcaagatacggcgagggggcttactggggccaagggactctggtcactgtctctgcagccaaaaca acagccccatcggtctatccactggcccctgtgtgtggagatacaagtggctcctcggtgactctaggatgcctggtcaag ggttatttccctgagccagtgaccttgacctggaactctggatccctgtccagtggtgtgcacaccttcccagctgtcctgca gtctgacctctacaccctcagcagctcagtgactgtaacctcgagcacctggcccagccagaccatcacctgcaatgtgct cgct |
| 45 | 22-4B11-B9 LCVR | gcatcgccttaacacgctcgtccttatttgctacactgaaggtgcccgtacgctttcgggtatcgttgacatgaaatgcccagt catgccgttggggtaagcgttgaccactgaagcttgacagttaagcctttgggtaatcgttgaacaataaaagatgccagttt agcctttggggcttttatttatagggcgggaaaacactagtgcctgttaggctgttggtgctctggattcgggaaaccaacgg tgatgttgtgatgacccagattccactcactttgtcggttatcattggacaaccagcctccatctcttgcaagtcaagtcagag cctcttatatagtgatggaaagacatatttgaattggttgttacagaggccaggccagtctccaaagcgcctaatctatctggt gtctaaactggactctggagtctctgacaggttcactggcagtggatcagggacagatttcacactgaaaatcagcagagt ggaggctgaggatttgggagtttattattgctggcaaggtacacattttccgcagacgtacggtggaggcaccaagctgga aatcaaacgggctgatgctgcaccaactgtatccatcttcccaccatccagtgagcagttaacatctggaggtgcctcagtc gtgtgcttcttgaacaacttctaccccaaagacatcaatgtcaagtggaagattgatggcagtgaacgacaaaatggcgtcc tgaacagttggactgatcaggacagcaaagacagcacctacagcatgagcagcaccctcacgttgaccaaggacgagta tgaacgacataacagctatacctggaggcccgcgtcgacacatcagcgccacctcagtgccaaggg |
| 46 | 22-4B11-B9 HCVR | acggtaatgaaagaagggggtggacctgggttttttttttgccgttttttttccaccttttttaaaatttggggttaaaaccccccc cctttttcaatgtagaacctgggctttattttaatcctgtcagtaactacaggtgtccactctgaggtccagctgcaacagtctg gacctgagctggtgaagcctggggcgtcagtgaagatttcctgcaaggcttctggttactcattcactggttttcctcatgcac tgggtgaagcaaagtcctgaaaagagccttgagtggattggagagattaatcctagtactggtggtactacctacaatcaga ggttcaaggccaaggccacattgactgtagacaaatcctacagtacagcctatatgcaactcaggagcctgacatctgaag actctgcagtctattactgtgcaagatacggcgagggggcttactggggccaagggactctggtctctgtctctgcggcca aaacaacagccccatcggtctatccactggcccctgtgtgtggagatacaagtggctcctcggtgactctaggatgcctgg tcaagggttatttccctgagccagtgaccttgacctggaactctggatccctgtccagtggtgtgcacaccttcccagctgtc ctgcagtctgacctctacaccctcagcagctcagtgactgtaacctcgagcacctggcccagccagtccatcacctgcatat aatcttgcaag |
| 47 | 22-5B1-B2 LCVR | ctgttcatgcctgcagacatgacagtcagaagatgagttacatcatcgagaatgaatagcttatgatattcgattgttcgataa caatgcatgacaaacaagctagtatgattgatcctacggtcactcagatggttactatgcaagacgaaagaagacagatgct acatatcaaactctcttggtgcaggtcatttacttccttttttgatgagtactttgcataaagttcctagagggcagaccagctgtt ccatgatttataaacctaggcccttctccacggagatctcaaaagcatcataacttcactgggggggcccccctcaaatgt gggggggggggaggacgccttatatccttgctgttctggttatatggtgctgatgggaacattgtaatgacccaatctcccaa atccatgtccatgtcagtaggagagagggtcaccttgagctgcaaggccagtgagaatgtgggtacttatgtatcctggtat caacagaaaccagagcagtctcctaaactgctgatatacggggcatccaaccggtacactggggtccccgatcgcttcac aggcagtggatcagcaacagatttcactctgaccatcagcagtgtgcaggctgaagaccttgcagattatcactgtggaca gagttacagctatccgctcacgttcggtgctgggaccaagctggagctgaaacgggctgatgctgcaccaactgtatccat cttcccaccatccagtgagcagttaacatctggaggtgcctcagtcgtgtgcttcttgaacaacttctaccccaaagaccatc aaagtcaaagtggaagattgatggcagtgaacgacaaaatggcgtcctgaacagttggactgatcaggacagcaaagac agcacctacagcatgagcagcaccctcacgttgaccaaggacgagtatgaacgacataacagctatacctgtgaggcca ctcacaagacatcaacttcacccattcaagg |
| 48 | 22-5B1-B2 HCVR | gtattatgtgaggtgtcttttcctggtggcatcttcatcctattgtcttctgtcaacgtgattttcttttatacagcttctgtactcagt atttatatattagtatgcatttatttctttcttatctgttttcgtaattacgtggggttaatcggactgttagaaagctgtgttgagattt gctttttatgtttgctttaattaggatgctattgatctgagtttgatatgtaattatacagcatccatttcgaatggtaacagtaagct cttatggatttcgacaggattcatgtacaaaggttttagcaatggtgcttcctgggttctatgctggagtctcttgttgtaattggt ggaagatttttaaacattcgctaaaaagcagcaaagaataataagaaaaaggtgttgaactgggtctttgttttaatcctgtca gtaactacaggtgtccactctgaggtccagatgcagcagtctggacctgagctggtgaagcctggggcttcagtggagatt tcctgcaaggcttctggttactcattcactggctattacatacactgggtgaagcaaagtcctgaaaagaaccttgaatggatt ggagaggttaatcctattagtggtggtactacctacaaccagagattcaaggccaaggccacattgaatgtggacacattat ccaacacagcctacatgcagctcaagagcctgacatctgaggactctgcagtctatttctgtgcacgatacggggctacgg actattggggtcaaggaacctcagtcaccgtctcctcagccaaaacaacagccccatcggtctatccactggcccctgtgt gtggagatacaagtggctcctcggtgactctaggatgcctggtcaagggttatttccctgagccagtgaccttgacctggaa ctctggatccctgtccagtggtgtgcacaccttcccagctgtcctgcagtctgacctctacaccctcagcagctcagtgactg taacctcgagcacctggcccagccagtccatctaccgctatagaacgtgca |
| 49 | 22-5F2-E10 HCVR | ttacaaaataaacagataagcgtttccattaaacataaaaaatttatggtaacgcttttttttttgggggggggggaaccaagtt gcctgttaggctgttggtgctctggattcgggataccaacggtgatgttgtgctgacccagactccactcactttgtcggttac ctttggacaaccagcctccatctcttgcaagtcaagtcagagcctctttatatagtgatggaaagacatatttgtatttggttgtt acagaggccaggccagtctccaaagcgcctaatctttctggtgtctaaactggactctggagtccctgacaggttcagtgg cagtggatcagggacagatttcacactgaagatcaacagagtggaggctgaggatttgggaatttattattgctggcaaggt acacattttcctcagacgtacggtggaggcaccatgctggaaatcaaacgggctgatgctgcaccaactgtatccatcttcc caccatccagtgagcagttaacatctggaggtgcctcagtcgtgtgcttcttgaacaacttctaccccaaagacatcaatgtc aagtggaagattgatggcagtgaacgacaaaatggcgtcctgaacagttggactgatcaggacagcaaagacagcacct acagcatgagcagcaccctcacgttgaccaaggacgagtatgaacgacataacagctatacctgtgaggtcactcacaag actatcaacttcacccattcaagtg |
| 50 | 22-5F2-E10 LCVR | acatacaatcaatcgaacatacacaaaacattacccctcttataaaacatttcgttacacctccctactcccccccgatgagtc gccgggctttattttaaatcctgtcagtaactacaggtgtccactctgaggtccagctgcagcagtctggacctgaactggtg aagcctggggcttcagtgaagatatcttgcaaggcttctggttactcattcactggctactacatacactgggtgaggcgaa gtcctgaaaagagccttgcgtggattggagagattaatcctaccactggtggtacttcctacaaccagaaattcagggccaa ggccacattgactgtagacacgtcgtccagcacagcctatattcagctcaagagcctgacatctgaggactctgcaatctat tactgtgcaagatacggcgagggggcttactggggccaagggactctggtcactgtctctgcagccaaaacaacagccc catcggtctatccactggcccctgtgtgtggagatacaagtgggctccctcggtgactctaggatgcctggtcaagggttatt tccctgagccagtgaccttgacctggaactctggatccctgtccagtggtgtgcacaccttcccagctgtcctgcagtctga cctctacaccctcagcagctcagtgactgtaacctcgagcacctggcccagccagtccatcacctcatacgacctgctaa |

Certain exemplary embodiments according to the present disclosure are described as below.

### Recombinant anti-GM2AP antibody or the antigen-binding fragment thereof

According to one embodiment of the present disclosure, the present application provides a recombinant antibody which specifically binds GM2-activator protein (GM2AP) (the "anti-GM2AP antibody") or the antigen-binding fragment thereof. The GM2AP of this embodiment may comprise an amino acid sequence of SEQ ID NO: 1, SEQ ID NO: 2 or SEQ ID NO: 3, or SEQ ID NO: 4. The anti-GM2AP antibody or the antigen-binding fragment thereof of the present embodiment may specifically bind to one or more amino acid residues within amino acids 1-14 of SEQ ID NO: 1 or the amino acids 57-70 of SEQ ID NO: 2 or SEQ ID NO: 3. Preferably, the anti-GM2AP antibody or the antigen-binding fragment thereof may specifically bind to GM2AP among amino acids 57-70 of SEQ ID NO:2 or SEQ ID NO: 3. In other words, said amino acids 1-14 of SEQ ID NO: 1 and amino acids 57-70 of SEQ ID NO: 2 or SEQ ID NO: 3 form the epitope of the anti-GM2AP antibody or the antigen-binding fragment thereof of the present embodiment.

As used herein, an antibody that "specifically binds to GM2AP" is intended to refer to an antibody that binds to GM2AP with a binding value KD of less than 1× 10⁻⁸ M, less than 1 × 10⁻⁹ M, less than 1 × 10⁻¹⁰ M, less than 1 × 10⁻¹¹, or even less.

The anti-GM2AP antibody or the antigen-binding fragment thereof may comprise a light chain variable region (LCVR) and a heavy chain variable region (HCVR). In some embodiments, the LCVR may comprise an amino acid sequence having at least 80%, 85%, 90%, 91%, 92%, 93%, 94%, 95%, 96%, 97, 98, 99%, or 100% (or any percentage in between) identity to SEQ ID NO: 5, 7, 9, 11, 13, 15, 17, 20, 22, 24 or 26. Meanwhile, the HCVR may preferably comprise an amino acid sequence having at least 80%, 85%, 90%, 91%, 92%, 93%, 94%, 95%, 96%, 97, 98, 99%, or 100% (or any percentage in between) identity to SEQ ID NO: 6, 8, 10, 12, 14, 16, 18, 19, 21, 23, 25 or 27. More preferably, the pair of LCVR and the HCVR may comprise the amino acid sequence pairs having at least 80%, 85%, 90%, 91%, 92%, 93%, 94%, 95%, 96%, 97, 98, 99%, or 100% (or any percentage in between) identity to SEQ ID NOs: 5/6, 7/8, 9/10, 11/12, 13/14, 15/16, 17/18, 17/19, 20/21; 22/23, 24/25 or 26/27, respectively.

Moreover, said LCVR may comprise three light chain complementary determining regions (LCDR1, LCDR2 and LCDR3). Each of LCDR1, LCDR2 and LCDR3 may comprise an amino acid sequence having at least 80%, 85%, 90%, 91%, 92%, 93%, 94%, 95%, 96%, 97, 98, 99%, or 100% (or any percentage in between) identity to SEQ ID NO: 51, 52, 53, 57, 58, 59, 63, 64, 65, 69, 70, 71, 75, 76, 77, 81, 82, 83, 87, 88, 89, 96, 97, 98, 102, 103, 104, 108, 109, 110, 114, 115, or 116. Meanwhile, said HCVR may comprise three heavy chain complementary determining regions (HCDR1, HCDR2, and HCDR3). Each of HCDR1, HCDR2 and HCDR3 may comprise an amino acid sequence having at least 80%, 85%, 90%, 91%, 92%, 93%, 94%, 95%, 96%, 97, 98, 99%, or 100% (or any percentage in between) identity to SEQ ID NO: 54, 55, 56, 60, 61, 62, 66, 67, 68, 72, 73, 74, 78, 79, 80, 84, 85, 86, 90, 91, 92, 93, 94, 95, 99, 100, 101, 105, 106, 107, 111, 112, 113, 117, 118 or 119.

More preferably, the LCDR1/LCDR2/LCDR3 may have the amino acid sequences having at least 80%, 85%, 90%, 91%, 92%, 93%, 94%, 95%, 96%, 97, 98, 99%, or 100% (or any percentage in between) identity to SEQ ID NOs: 51/52/53, 57/58/59, 63/64/65, 69/70/71, 75/76/77, 81/82/83, 87/88/89, 96/97/98, 102/103/104, 108/109/110 or 114/115/116, respectively. In addition, the HCDR1/HCDR2/HCDR3 may have the amino acid sequences having at least 80%, 85%, 90%, 91%, 92%, 93%, 94%, 95%, 96%, 97, 98, 99%, or 100% (or any percentage in between) identity to SEQ ID NOs: 54/55/56, 60/61/62, 66/67/68, 72/73/74, 78/79/80, 84/85/86, 90/91/92, 93/94/95, 99/100/101, 105/106/107, 111/112/113 or 117/118/119, respectively. Still more preferably, the LCDR1/LCDR2/LCDR3/HCDR1/HCDR2/HCDR3 may have amino acid sequences having at least 80%, 85%, 90%, 91%, 92%, 93%, 94%, 95%, 96%, 97, 98, 99%, or 100% (or any percentage in between) identity to SEQ ID NOs: 51/52/53/54/55/56, 57/58/59/60/61/62, 63/64/65/66/67/68, 69/70/71/72/73/74, 75/76/77/78/79/80, 81/82/83/84/85/86, 87/88/89/90/91/92, 87/88/89/93/94/95, 96/97/98/99/100/101, 102/103/104/105/106/107, 108/109/110/111/112/113, or 114/115/116/117/118/119, respectively.

The anti-GM2AP antibody or the antigen-binding fragment thereof, as used herein, can be a diabody, a Fab, a Fab', a F(ab')₂, an Fv fragment, a disulfide stabilized Fv fragment (dsFv), a (dsFv)₂, a bispecific dsFv (dsfv-dsfv'), a disulfide stabilized diabody (ds diabody), a single-chain antibody molecule (scfv), an scfv dimer (bivalent diabody) or any combination thereof. Moreover, the anti-GM2AP antibody or the antigen-binding fragment thereof can be a monoclonal antibody, multispecific antibody, human antibody, humanized antibody, chimeric antibody and any combination thereof. Preferably, the recombinant antibody or the antigen-binding fragment thereof is a human antibody.

### Polynucleotides encoding the anti-GM2AP antibody or the antigen-binding fragment thereof, and vectors and host cells comprising the same

According to one embodiment of this disclosure, the present application also provides a polynucleotide encoding any one of the anti-GM2AP antibodies or the antigen-binding fragments thereof mentioned in the present disclosure. The polynucleotide may comprise a nucleic acid sequence having at least 80%, 85%, 90%, 91%, 92%, 93%, 94%, 95%, 96%, 97, 98, 99%, or 100% (or any percentage in between) identity to SEQ ID NOs: 28-50. In some embodiment, the nucleic acid sequence encoding the LCVR of the recombinant antibody of the foregoing embodiment or the antigen-binding fragment thereof may have at least 80%, 85%, 90%, 91%, 92%, 93%, 94%, 95%, 96%, 97, 98, 99%, or 100% (or any percentage in between) identity to SEQ ID NOs: 28, 30, 32, 34, 36, 38, 40, 43, 45, 47 or 50. Meanwhile, the nucleic acid sequence encoding the HCVR of the recombinant antibody of the foregoing embodiment or the antigen-binding fragment thereof may have at least 80%, 85%, 90%, 91%, 92%, 93%, 94%, 95%, 96%, 97, 98, 99%, or 100% (or any percentage in between) identity to SEQ ID NOs: 29, 31, 33, 35, 37, 39, 41, 42, 44, 46, 48 or 49.

Moreover, the LCVR/HCVR pair of the anti-GM2AP antibody of the foregoing embodiment or the antigen-binding fragment thereof may be encoded by the nucleic acid sequence pair having at least 80%, 85%, 90%, 91%, 92%, 93%, 94%, 95%, 96%, 97, 98, 99%, or 100% (or any percentage in between) identity to SEQ ID NOs: 28/29, 30/31, 32/33, 34/35, 36/37, 38/39, 40/41, 40/42, 43/44, 45/46, 47/48, or 50/49, respectively.

In some embodiments, the polynucleotides are substantially identical to those coding for the amino acid sequences as described above. Substantially identical sequences may be polymorphic sequences, i.e., alternative sequences or alleles in a population. Substantially identical sequences may also comprise mutagenized sequences, including sequences comprising silent mutations. A mutation may comprise one or more nucleotide residue changes, a deletion of one or more nucleotide residues, or an insertion of one or more additional nucleotide residues. Substantially identical sequences may also comprise various nucleotide sequences that encode for the same amino acid at any given amino acid position in an amino acid sequence disclosed herein, due to the degeneracy of the nucleic acid code.

For the purpose of this disclosure, a vector comprising the polynucleotides as described above is also provided. Moreover, a host cell comprising the vector as described above is also provided. In some embodiment, the host cell can be a eukaryotic cell, preferably artificially selected or genetically engineered. The eukaryotic cell may comprise, but not be limited to, a Chinese Hamster Ovary (CHO) cell, NSO cell, BHK cell, SP2/0 cell, HEK 293 cell, HEK 293 EBNA cell, PER.C6^{®} cell, COS cell, 239F cell, SF9 cell, SF21 cell and a combination thereof.

In general, the polynucleotides, vectors and host cells comprising the same can be used to generate the anti-GM2AP antibodies or the antigen-binding fragment thereof provided by the aforementioned embodiments. More detailed, the polynucleotides of this embodiment can be, for example, DNA or RNA and may or may not contain intronic sequences. Preferably, the polynucleotides can be a cDNA molecule.

The polynucleotides of this embodiment can be isolated and obtained by any method known in the art. For example, if the nucleotide sequence of an antibody is known, a polynucleotide encoding the antibody may be assembled from chemically synthesized oligonucleotides. This would involve, for example, the synthesis of overlapping oligonucleotides containing portions of the sequence encoding the antibody, annealing and ligating those oligonucleotides, and then amplifying the ligated oligonucleotides by PCR. The disclosed polynucleotides can also be generated from any other suitable source of nucleic acids, such as an antibody cDNA library, or a cDNA library isolated from any tissue or cells expressing the antibody (e.g., from hybridoma cells selected to express an antibody). The cDNAs encoding the light and heavy chains of the anti-GM2AP antibody made by a host cell (e.g., a hybridoma) can be obtained by standard PCR amplification or cDNA cloning techniques. For antibodies obtained from using phage display techniques, the polynucleotides encoding the anti-GM2AP antibodies provided by the aforementioned embodiments can be recovered from various phage clones of the library. In some embodiments, any of the disclosed polynucleotides or isolated nucleic acids may be incorporated into an expression vector. Suitable vectors for expression in various human and animal cell types are known in the art. In some embodiments, host cells are provided comprising the vectors. Suitable host cells include, e.g., a Chinese Hamster Ovary (CHO) cell, NSO cell, BHK cell, SP2/0 cell, HEK 293 cell, HEK 293 EBNA cell, PER.C6^{®} cell, COS cell, 239F cell, SF9 cell, SF21 cell, and/or other human or nonhuman cell lines. In some embodiments, the host cells may transiently or stably express the polynucleotides or the isolated nucleic acids on the vector when cultured in culture medium, thereby providing a method for producing the antibodies or fragments disclosed herein.

Once the DNA fragments encoding V_{H} and V_{L} segments are obtained, these DNA fragments can be further manipulated by standard recombinant DNA techniques, for example to convert the variable region genes to full-length antibody chain genes, to Fab fragment genes or to an scFv gene. In these manipulations, a V_{L}- or V_{H}-encoding DNA fragment is operatively linked to another DNA molecule, or to a fragment encoding another protein, such as an antibody constant region or a flexible linker. The term "operatively linked", as used in this context, is intended to mean that the two DNA fragments are joined in a functional manner, for example, such that the amino acid sequences encoded by the two DNA fragments remain in-frame, or such that the protein is expressed under control of a desired promoter.

The isolated DNA encoding the V_{H} region can be converted to a full-length heavy chain gene by operatively linking the V_{H}-encoding DNA to another DNA molecule encoding heavy chain constant regions (CH1, CH2 and CH3). The sequences of mouse and human (or other mammals') heavy chain constant region genes are known in the art (see e.g., Kabat, E. A., et al., (1991) Sequences of Proteins of Immunological Interest, Fifth Edition, U. S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. In some embodiments, the heavy chain constant region is selected among IgG1 isotypes. For a Fab fragment heavy chain gene, the VH-encoding DNA can be operatively linked to another DNA molecule encoding only the heavy chain CH1 constant region.

The isolated DNA encoding the V_{L} region can be converted to a full-length light chain gene (as well as to a Fab light chain gene) by operatively linking the V_{L}-encoding DNA to another DNA molecule encoding the light chain constant region, CL. The sequences of mouse and human (or other mammals') light chain constant region genes are known in the art (see e.g., Kabat, E. A., et al., (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or a lambda constant region.

To create an scFv gene, the V_{H}- and V_{L}-encoding DNA fragments are operatively linked to another fragment encoding a flexible linker, e.g., encoding the amino acid sequence (Gly4-Ser)₃ such that the V_{H} and V_{L} sequences can be expressed as a contiguous single-chain protein, with the V_{L} and V_{H} regions joined by the flexible linker (see e.g., Bird et al., 1988 Science 242:423-426; Huston et at., (1988) Proc. Natl. Acad. Sci. USA 85:5879-5883; McCafferty et al., (1990) Nature 348:552-554).

### Kits for detecting GM2AP

According to one embodiment of the present disclosure, the present application also provides a kit for detecting a GM2-activator protein (GM2AP) in a biological sample. The kit may comprise the anti-GM2AP antibody or the antigen-binding fragment thereof mentioned in the present disclosure. Preferably, the kit of this embodiment may further comprise a substrate and a second antibody which conjugates with a signal generating unit and binds to a Fc domain of the anti-GM2AP antibody or the antigen-binding fragment thereof. The substrate is configured to react with the signal generating unit to generate a signal. Moreover, the kit may further comprise a blocking solution and/or a wash solution. Preferably, the blocking solution may comprise skim milk, bovine serum albumin, or casein and the wash solution may comprise PBS, TBS, PBS including a detergent, or TBS including a detergent.

In some embodiments, the biological sample may comprise whole blood, serum, plasma, urine, or a combination thereof. In addition, the signal generating unit may comprise a radioactive marker, a fluorescent marker, a phosphorescent marker, a chemiluminescent marker or a labeling enzyme. Preferably, the labeling enzyme may comprise a horse radish peroxidase or an alkaline phosphatase.

More preferably, the substrate may be tetramethylbenzidine (TMB) provided that the signal generating unit is a horse radish peroxidase.

### Methods for detecting GM2AP

The present application also provides a method for detecting a GM2-activator protein (GM2AP) in a biological sample by the foregoing anti-GM2AP antibody or the antigen-binding fragment thereof, or more specifically, by the kit of the preceding embodiment. The method of this embodiment may the following steps:
(a) applying the anti-GM2AP or the antigen-binding fragment thereof mentioned in the present disclosure to said biological sample;
(b) incubating said biological sample with a secondary antibody conjugated with a detectable label; and
(c) detecting said detectable label.

For the purpose of the present disclosure, the biological sample may comprise whole blood, serum, plasma, urine, or a combination thereof, and the secondary antibody may bind to a Fc domain of said anti-GM2AP antibody or the antigen-binding fragment thereof. Moreover, the detectable label may comprise an alkaline phosphatase. Meanwhile, the step (c) can be conducted by incubating said detectable label with a *p*-nitrophenyl phosphate solution and determining an absorbance at OD₄₀₅.

Alternatively, the detectable label may comprises a horse radish peroxidase and the step (c) can be conducted by incubating said detectable label with a tetramethylbenzidine (TMB) solution and determining an absorbance at OD₄₅₀.

### Methods for inducing anti-GM2AP antibody or the antigen-binding fragment thereof

The present application also provides a method for inducing any one of the anti-GM2AP antibody or the antigen-binding fragment thereof mentioned in the present disclosure. The method of this embodiment may comprise the following steps:
(a) administering to a subject a peptide fragment comprising any one of the amino acid sequences of SEQ ID NOs: 1 to 4 listed in Table 1;
(b) forming hybridoma cells; and
(c) screening for a hybridoma cell secreting the antibody specifically binding to the glycosylated GM2AP.

Preferably, said peptide fragment of step (a) may comprises the amino acid sequence of SEQ ID NO: 1.

It will be apparent to those skilled in the art that various modifications and variations can be made to the disclosed embodiments. It is intended that the following experimental examples are considered as exemplary only.

### Experimental Examples

All materials used in the following experimental examples are commercially available or can be easily obtained by the person with ordinary skills in the art from related commercially available materials without undue experiments.

### Production and purification of GM2AP

In the present application, GM2AP or a peptide fragment of the GM2AP was i.p. injected into BALB/C mice to generate monoclonal antibodies. Specifically, in an embodiment, a peptide fragment of the GM2AP (SEQ ID NO: 1, PIIVPGNVTLSVVG) linked with (GlcNAc)₂Fuc(Man)₃ was used to induce antibodies in a test subject, such as mice. In another embodiment, the GM2AP (SEQ ID NO: 2 or SEQ ID NO: 3) linked with (GlcNAc)₂Fuc(Man)₃ was used to induce antibodies in a test subject. In still another embodiment, the GM2AP tagged with poly(His), such as eight Histidines, (SEQ ID NO: 4) was used to induce antibodies in a test subject for the convenience of purification. Other tags, such as FLAG, Myc, HA, etc., may be applied as well.

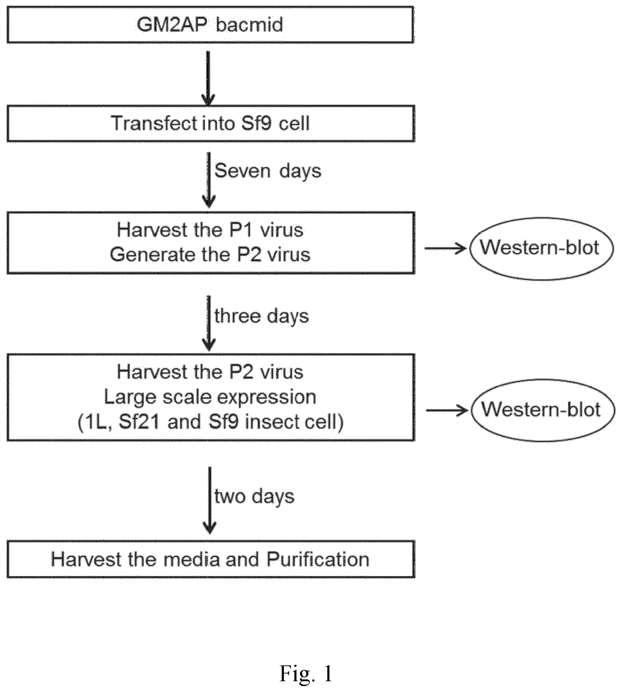

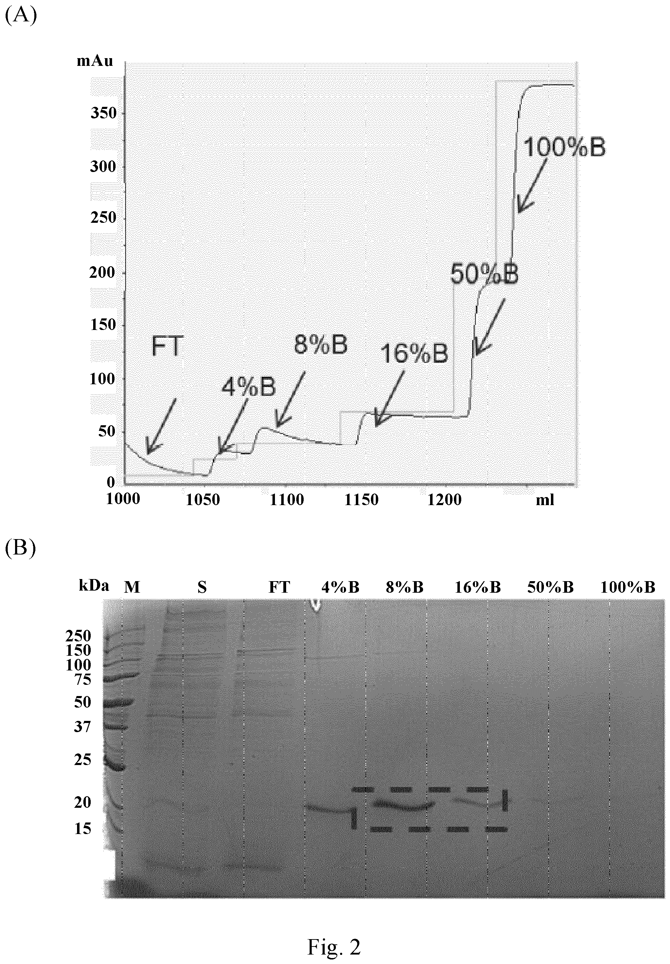

To generate monoclonal antibodies specifically recognizing GM2AP, briefly, the recombinant GM2AP (SEQ ID NO: 4) was expressed in SF9 cells and/or SF21 cells (FIG. 1). The expressed recombinant GM2AP was purified by Ni Excell column (Buffer A: 0.3M NaCl, 25mM sodium phosphate, 20mM Imidazole, pH8.0; Buffer B: 0.3M NaCl, 25mM sodium phosphate, 500mM Imidazole, pH8.0; dialysis Buffer: 0.3M NaCl, 25mM sodium phosphate, pH8.0). The SF21 cell lysate was first filtered through 0.22uM membrane. The Ni Excell column was pre-equilibrated with Buffer A followed by loading the filtered cell lysate onto column. The column was washed with Buffer A and the recombinant GM2AP was eluted from the column with 4%, 8%, 16%, 50% and 100% of buffer B. The 16% and 8% fractions were harvested and dialyzed in dialysis Buffer (FIG. 2).

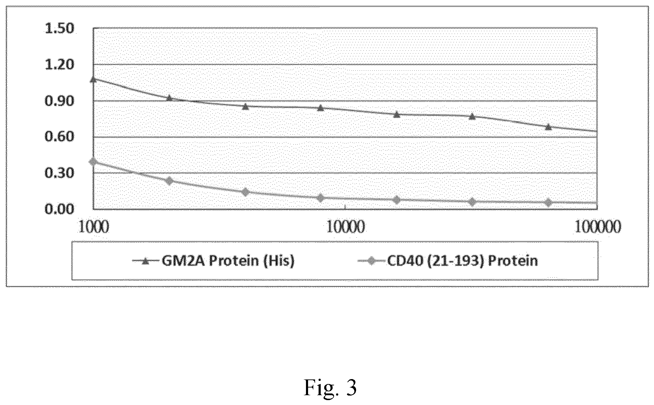

The purified GM2AP was i.p. injected into BALB/C mice to generate monoclonal antibodies. A titer of the generated anti-GM2AP serum was tested by indirect ELISA. Briefly, GM2A protein (His-tagged) and CD40 (21-193) protein (negative control) were coated on the ELISA plate. Mouse Anti-Histidine Tag mAb (1000X) was used as positive control and normal sera were used as negative control. D56 and D31 pre-bleeding sera were subject to serial dilution and added into the coated wells. Goat Anti-Mouse IgG-HRP (1500X) was used as secondary antibody. The absorbance OD₄₅₀ of the sample mixtures was measured. The results were shown in Tables 4 and 5, and FIG. 3.

Afterwards, the mice were sacrificed and the activated splenocytes were fused with melanoma cells to generate hybridomas. The hybridoma clones which showed the highest affinities to the GM2AP among all were i.p. injected into mice for ascites production to amplify the antibodies. The antibody clones which showed the highest affinity to GM2AP among all were further selected for further investigation, for example, clone No. 21-2B5-F7, 21-2H9-G2, 21-4E3-C8, 21-4B10-E1, 21-4G4-B11, 22-5B6-B2, 22-5F2-E10, 22-4B11-B9, 22-3E6-B11, 22-2E9-G12, etc.

### Statistical analysis

All data were shown as mean ± STDEV. The data comparison was determined by unpaired Student *t* test or ANOVA. The *p* values less than 0.05 were considered as statistically significant.

### Results

As shown Tables 4 and 5, and FIG. 3, both the D56 and D31 pre-bleeding sera comprises the antibodies specifically binding to GM2AP, instead CD40 (21-193) protein. The presence of anti-GM2AP antibodies was further confirmed by western blot (data not shown).

**Table 4. Titer of anti-GM2AP serum_D56**

| Mouse No100352622 | GM2AP (His) | CD40 (21-193) Protein |
|---|---|---|
| positive control (PC) | 1.02 | 1.08 |
| Sera dilution in 1000X | 1.08 | 0.39 |
| Sera dilution in 2000X | 0.92 | 0.24 |
| Sera dilution in 4000X | 0.86 | 0.14 |
| Sera dilution in 8000X | 0.84 | 0.10 |
| Sera dilution in 16000X | 0.79 | 0.08 |
| Sera dilution in 32000X | 0.77 | 0.06 |
| Sera dilution in 64000X | 0.69 | 0.06 |
| Sera dilution in 128000X | 0.62 | 0.05 |
| Sera dilution in 256000X | 0.52 | 0.05 |
| Sera dilution in 512000X | 0.38 | 0.05 |
| Sera dilution in 1024000X | 0.22 | 0.04 |

**Table 5. Titer of anti-GM2AP serum_D31**

| Mouse No 100352622 | GM2AP (His) | CD40 (21-193) Protein |
|---|---|---|
| positive control (PC) | 1.02 | 1.08 |
| Sera dilution in 1000X | 1.03 | 0.06 |
| Sera dilution in 16000X | 0.54 | 0.05 |
| Sera dilution in 1024000X | 0.12 | 0.05 |

In addition, the antibody clones which showed the highest affinity to GM2AP among all were further selected for further investigation. That is, antibodies of clone No. 21-2B5-F7, 21-2H9-G2, 21-4E3-C8, 21-4B10-E1, 21-4G4-B11, 22-5B6-B2, 22-5F2-E10, 22-4B11-B9, 22-3E6-B11 and 22-2E9-G12 were subject to sequence analysis. The amino acid sequences of the LCVR/HCVR and the corresponding CDRs (LCDR1-3 and HCDR1-3) are shown in Table 2. The nucleic acids sequences encoding the LCVR/HCVR of these monoclonal antibodies are shown in Table 3. In addition, the concentrations of said monoclonal antibodies are listed below.

**Table 6. Concentration of monoclonal anti-GM2AP antibodies**

| No. | Antibody No. | Label Conc. (mg/mL) | Measurement Conc. (mg/mL) |
|---|---|---|---|
| GM1 | 22-5F2-E10 | 3.67 | 3.72 |
| GM2 | 22-5B6-B2 | 3.58 | 3.41 |
| GM3 | 22-4B11-B9 | 3.11 | 3.12 |
| GM4 | 22-3E6-B11 | 3.23 | 3.18 |
| GM5 | 22-2E9-G12 | 3.1 | 3.42 |
| GM6 | 21-4B10-E1 | 3.96 | 4.04 |
| GM7 | 21-4G4-B11 | 2.5 | 2.9 |
| GM8 | 21-2H9-G2 | 3.34 | 3.38 |
| GM9 | 21-4E3-C8 | 2.25 | 2.57 |
| GM10 | 21-2B5-F7 | 2.64 | 2.83 |

### Comparison of the anti-GM2AP antibodies of the present application between other commercial ELISA kits by Western Blot assay

Four of the antibody clones (21-4B10-E1, 21-4E3-C8, 21-2H9-G2, and 21-2B5-F7) provided by the preceding examples are compared with one commercial anti-GM2AP antibody (Sigma) by Western Blot assay. The assay was carried out by the following steps.

A 12 mL urine sample obtained from a test subject was centrifuged with a 50kDa cut-off Centricon centrifugal filter device (Millipore) until the volume of the sample in the upper part is less than 500 µL. Deionized water was added into the upper part to a final volume of 12 mL, followed by centrifugation, until the volume of the sample in the upper part is less than 500 µL. Deionized water was added into the upper part for a second time to a final volume of 12 mL, followed by centrifugation, until the volume of the sample in the upper part is less than 500 µL. The collected sample was subject to desalting and concentration, followed by SDS-PAGE without reducing agent in sample buffer. For the SDS-PAGE, each well was loaded with 25 µL of the collected sample (about 10 µg of protein in amount). The gel after running was transferred to a nitrocellulose (NC) membrane, followed by Western Blot assay stained with four clones of anti-GM2AP antibodies (21-4B10-E1, 21-4E3-C8, 21-2H9-G2, and 21-2B5-F7) provided by the preceding example and the commercial anti-GM2AP antibody (Sigma) as the first antibodies and with suitable secondary anti-Fc antibodies through the standard protocol in the lab. The commercial anti-GM2AP antibody (Sigma) was diluted in 1:1000 before use. The concentrations of the four anti-GM2AP antibodies were summarized in Table 7.

**Table 7. Concentration of the anti-GM2AP antibodies for Western Blot**

| No. | Antibody No. | Conc. (mg/mL) |
|---|---|---|
| GM6 | 21-4B10-E1 | 2.6 |
| GM8 | 21-2H9-G2 | 1.7 |
| GM9 | 21-4E3-C8 | 3.53 |
| GM10 | 21-2B5-F7 | 4.07 |

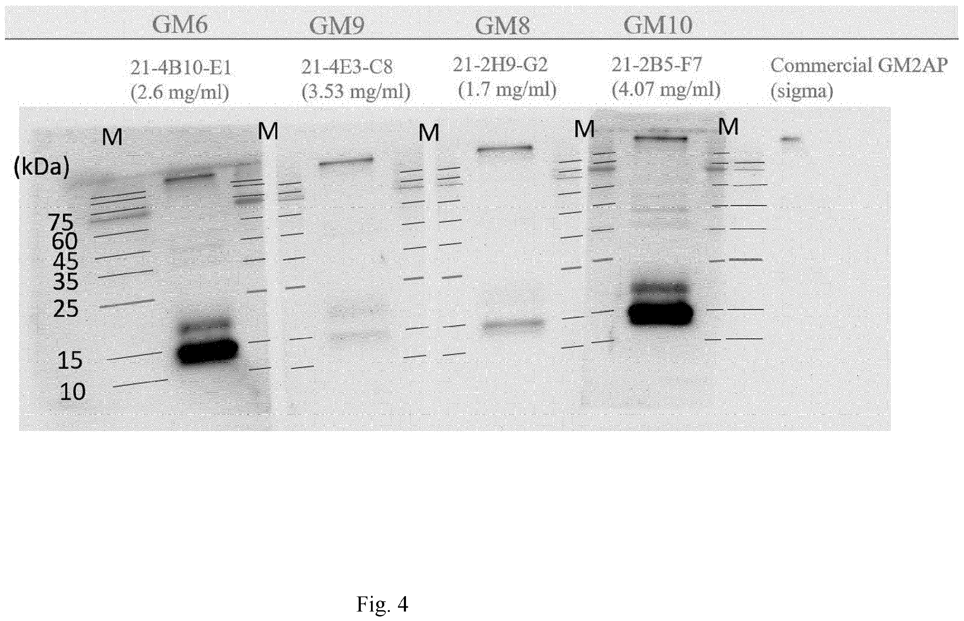

The result was demonstrated in Fig. 4. As shown in Fig. 4, it was obvious that the GM2AP protein in the urine sample was detected by all of the four anti-GM2AP antibodies clones provided by the preceding examples, whereas such signal was absent from the lane stained with the commercial anti-GM2AP antibody (Sigma).

### Sensitivity of the anti-GM2AP antibodies by ELISA and SPR assay

Various fragments (50 ng/well, Table 8) of GM2AP protein with N- or C-terminal biotin modification (C-terminal biotin modified fragments were designated with "(C)") in the wells of streptavidin plates. After incubation under 37°C for one hour, each well was added with 500 ng/well of first antibodies (GM9 = detection antibody; GM10 = capture antibody), followed by incubation under 37°C for another one hour. The secondary antibodies (anti-mouse (HRP), 1:10000 diluted) were added into each well and the plates were incubation under 37°C for yet another one hour. TMB was added as the substrate and coloring agent for reaction for 5 minutes in the dark. Stop solution was then added to quench the reaction and absorbance at OD₄₅₀ of each well was determined.

**Table 8. Fragments of VITAEin**

| No. | VITAEin |
|---|---|
| 1 | 1-30 (C) |
| 2 | 1-30 |
| 3 | 21-50 |
| 4 | 41-70 |
| 5 | 61-90 |
| 6 | 81-110 |
| 7 | 121-149 (C) |
| 8 | 121-150 |
| 9 | 141-170 |
| 10 | 161-193 |

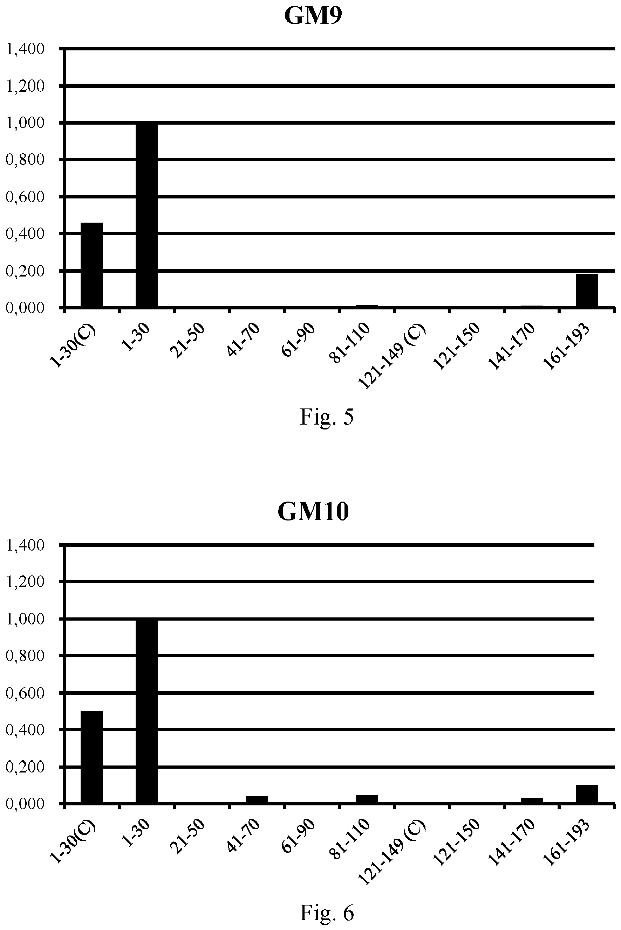

As shown in Figs. 5 and 6, GM9 and GM10 recognized the portion of VITAEin 1-30 significantly and the portion of GM2AP 170-193 mildly. After structural analysis (data not shown), VITAEin 1-30 and VITAEin 170-193 were positioned closely in structure. Hence, it is inferred that the antibodies can recognize the portion of VITAEin 1-30 and also recognize both the portions of VITAE1-30 and VITAEin 170-193 in spatial. VITAEin 1-30 and VITAE 170-193 were subject to Protein BLAST on NCBI. Non-VITAEin proteins were not present in the top 100 hits of the result. Hence, the antibodies were demonstrated to have high specificity toward the recognizing regions.

Moreover, the GM9 and GM10 antibody pair (GM10 = capture; GM9 = detection) were analyzed for their kₐ (M⁻¹s⁻¹), k_{d} (s⁻¹) and K_{D} (M) values by Surface plasmon resonance (SPR) assay with GE BIAcore 3000 instrument, as summarized in Table 9.

**Table 9 K_{D} values of the anti-GM2AP antibodies**

| Ab | kₐ (M⁻¹s⁻¹) | k_{d} (s⁻¹) | K_{D} (M) |
|---|---|---|---|
| GM9 | 3.26E5 | 4.09E-5 | 1.25E-10 |
| GM10 | 2.1E5 | 1.9E-4 | 8.8E-10 |

From the Table 9, GM9 and GM10 were demonstrated to have a K_{D} of 1.25E-10 M and 8.8E-10 M by SPR assay, respectively. Hence, the antibodies GM9 and GM10 were demonstrated to have a high affinity toward the antigen and were difficult to dissociate from the antigen.

Without further elaboration, it is believed that one skilled in the art can, based on the above description, utilize the present invention to its fullest extent. The specific examples provided herein are, therefore, to be construed as merely illustrative, and not limitative of the remainder of the disclosure in any way whatsoever.

All references (e.g., publications or patents or patent applications) cited herein are incorporated herein by reference in their entirety and for all purposes to the same extent as if each individual reference (e.g., publication or patent or patent application) was specifically and individually indicated to be incorporated by reference in its entirety for all purposes. Other embodiments are within the following claims.

## Claims

1. An recombinant antibody or the antigen-binding fragment thereof which specifically binds GM2-activator protein (GM2AP), wherein the recombinant antibody or the antigen-binding fragment thereof comprises:
a light chain variable region (LCVR) comprising three light chain complementary determining regions (LCDR1-3) amino acid sequences which are independently selected from the group consisting of SEQ ID NOs: 51-53, 57-59, 63-65, 69-71, 75-77, 81-83, 87-89, 96-98, 102-104, 108-110 and 114-116; and
a heavy chain variable region (HCVR) comprising three heavy chain complementary determining regions (HCDR1-3) amino acid sequences which are independently selected from the group consisting of SEQ ID NOs: 54-56, 60-62, 66-68, 72-74, 78-80, 84-86, 90-92, 93-95, 99-101, 105-107, 111-113, and 117-119.

2. The recombinant antibody or the antigen-binding fragment thereof according to claim 1, wherein the LCVR comprises an amino acid sequence which is selected from the group consisting of SEQ ID NOs: 5, 7, 9, 11, 13, 15, 17, 20, 22, 24 and 26.

3. The recombinant antibody or the antigen-binding fragment thereof according to claim 2, wherein the HCVR comprises an amino acid sequence which is selected from the group consisting of SEQ ID NOs: 6, 8, 10, 12, 14, 16, 18, 19, 21, 23, 25 and 27.

4. The recombinant antibody or the antigen-binding fragment thereof according to claim 3, comprising:
the LCVR comprising an amino acid sequence of SEQ ID NO: 5 and the HCVR comprising an amino acid sequence of SEQ ID NO: 6;
the LCVR comprising an amino acid sequence of SEQ ID NO: 7 and the HCVR comprising an amino acid sequence of SEQ ID NO: 8;
the LCVR comprising an amino acid sequence of SEQ ID NO: 9 and the HCVR comprising an amino acid sequence of SEQ ID NO: 10;
the LCVR comprising an amino acid sequence of SEQ ID NO: 11 and the HCVR comprising an amino acid sequence of SEQ ID NO: 12;
the LCVR comprising an amino acid sequence of SEQ ID NO: 13 and the HCVR comprising an amino acid sequence of SEQ ID NO: 14;
the LCVR comprising an amino acid sequence of SEQ ID NO: 15 and the HCVR comprising an amino acid sequence of SEQ ID NO: 16;
the LCVR comprising an amino acid sequence of SEQ ID NO: 17 and the HCVR comprising an amino acid sequence of SEQ ID NO: 18;
the LCVR comprising an amino acid sequence of SEQ ID NO: 17 and the HCVR comprising an amino acid sequence of SEQ ID NO: 19;
the LCVR comprising an amino acid sequence of SEQ ID NO: 20 and the HCVR comprising an amino acid sequence of SEQ ID NO: 21;
the LCVR comprising an amino acid sequence of SEQ ID NO: 22 and the HCVR comprising an amino acid sequence of SEQ ID NO: 23;
the LCVR comprising an amino acid sequence of SEQ ID NO: 24 and the HCVR comprising an amino acid sequence of SEQ ID NO: 25; or
the LCVR comprising an amino acid sequence of SEQ ID NO: 26 and the HCVR comprising an amino acid sequence of SEQ ID NO: 27.

5. The recombinant antibody or the antigen-binding fragment thereof according to claim 1, comprising LCDR1/LCDR2/LCDR3 amino acid sequences of SEQ ID NOs: 51/52/53, 57/58/59, 63/64/65, 69/70/71, 75/76/77, 81/82/83, 87/88/89, 96/97/98, 102/103/104, 108/109/110 or 114/115/116.

6. The recombinant antibody or the antigen-binding fragment thereof according to claim 2, comprising HCDR1/HCDR2/HCDR3 amino acid sequences of SEQ ID NOs: 54/55/56, 60/61/62, 66/67/68, 72/73/74, 78/79/80, 84/85/86, 90/91/92, 93/94/95, 99/100/101, 105/106/107, 111/112/113 or 117/118/119.

7. The recombinant antibody or the antigen-binding fragment thereof according to claim 3, comprising LCDR1/LCDR2/LCDR3/HCDR1/HCDR2/HCDR3 amino acid sequences of SEQ ID NOs: 51/52/53/54/55/56, 57/58/59/60/61/62, 63/64/65/66/67/68, 69/70/71/72/73/74, 75/76/77/78/79/80, 81/82/83/84/85/86, 87/88/89/90/91/92, 87/88/89/93/94/95, 96/97/98/99/100/101, 102/103/104/105/106/107, 108/109/110/111/112/113, or 114/115/116/117/118/119.

8. The recombinant antibody or the antigen-binding fragment thereof according to claim 1, wherein the recombinant antibody or the antigen-binding fragment thereof is selected from the group consisting of a diabody, a Fab, a Fab', a F(ab')₂, an Fv fragment, a disulfide stabilized Fv fragment (dsFv), a (dsFv)₂, a bispecific dsFv (dsfv-dsfv'), a disulfide stabilized diabody (ds diabody), a single-chain antibody molecule (scfv), an scfv dimer (bivalent diabody) and any combination thereof.

9. The recombinant antibody or the antigen-binding fragment thereof according to claim 1, wherein the recombinant antibody or the antigen-binding fragment thereof is selected from the group consisting of a monoclonal antibody, multispecific antibody, human antibody, humanized antibody, chimeric antibody and any combination thereof.

10. The recombinant antibody or the antigen-binding fragment thereof according to claim 1, wherein the recombinant antibody or the antigen-binding fragment thereof is a human antibody.

11. A polynucleotide encoding the recombinant antibody or the antigen-binding fragment thereof according to claim 1, comprising a nucleic acid sequence which is selected from the group consisting of SEQ ID NOs: 28-50.

12. The polynucleotide according to claim 11, wherein the nucleic acid sequence encoding the LCVR is selected from the group consisting of SEQ ID NOs: 28, 30, 32, 34, 36, 38, 40, 43, 45, 47 and 50.

13. The polynucleotide according to claim 12, wherein the nucleic acid sequence encoding the HCVR is selected from the group consisting of SEQ ID NOs: 29, 31, 33, 35, 37, 39, 41, 42, 44, 46, 48 and 49.

14. The polynucleotide according to claim 13, wherein:
the LCVR is encoded by the nucleic acid sequence of SEQ ID NO: 28 and the HCVR is encoded by the nucleic acid sequence of SEQ ID NO: 29;
the LCVR is encoded by the nucleic acid sequence of SEQ ID NO: 30 and the HCVR is encoded by the nucleic acid sequence of SEQ ID NO: 31;
the LCVR is encoded by the nucleic acid sequence of SEQ ID NO: 32 and the HCVR is encoded by the nucleic acid sequence of SEQ ID NO: 33;
the LCVR is encoded by the nucleic acid sequence of SEQ ID NO: 34 and the HCVR is encoded by the nucleic acid sequence of SEQ ID NO: 35;
the LCVR is encoded by the nucleic acid sequence of SEQ ID NO: 36 and the HCVR is encoded by the nucleic acid sequence of SEQ ID NO: 37;
the LCVR is encoded by the nucleic acid sequence of SEQ ID NO: 38 and the HCVR is encoded by the nucleic acid sequence of SEQ ID NO: 39;
the LCVR is encoded by the nucleic acid sequence of SEQ ID NO: 40 and the HCVR is encoded by the nucleic acid sequence of SEQ ID NO: 41;
the LCVR is encoded by the nucleic acid sequence of SEQ ID NO: 40 and the HCVR is encoded by the nucleic acid sequence of SEQ ID NO: 42;
the LCVR is encoded by the nucleic acid sequence of SEQ ID NO: 43 and the HCVR is encoded by the nucleic acid sequence of SEQ ID NO: 44;
the LCVR is encoded by the nucleic acid sequence of SEQ ID NO: 45 and the HCVR is encoded by the nucleic acid sequence of SEQ ID NO: 46;
the LCVR is encoded by the nucleic acid sequence of SEQ ID NO: 47 and the HCVR is encoded by the nucleic acid sequence of SEQ ID NO: 48; or
the LCVR is encoded by the nucleic acid sequence of SEQ ID NO: 50 and the HCVR is encoded by the nucleic acid sequence of SEQ ID NO: 49.

15. A method for detecting a GM2-activator protein (GM2AP) in a biological sample, comprising:
(a) applying the recombinant antibody or the antigen-binding fragment thereof according to claim 1 to said biological sample;
(b) incubating said biological sample with a secondary antibody conjugated with a detectable label; and
(c) detecting said detectable label;
optionally
wherein the biological sample comprises whole blood, serum, plasma, urine, or a combination thereof;
wherein the secondary antibody binds to a Fc domain of the antibody or the antigen-binding fragment thereof;
wherein the detectable label comprises an alkaline phosphatase;
wherein the step (c) is conducted by incubating said detectable label with a p-nitrophenyl phosphate solution and determining an absorbance at OD₄₀₅; or
wherein the step (c) is conducted by incubating said detectable label with a tetramethylbenzidine (TMB) solution and determining an absorbance at OD₄₅₀.
